(19) Europäisches Patentamt / European Patent Office / Office européen des brevets

(11) EP 4 659 748 A1

(12) EUROPEAN PATENT APPLICATION

(43) Date of publication:
**10.12.2025 Bulletin 2025/50**

(21) Application number: **24202826.4**

(22) Date of filing: **26.09.2024**

(51) International Patent Classification (IPC):
***A61K 31/352*** *(2006.01)* ***A61P 1/16*** *(2006.01)*

(52) Cooperative Patent Classification (CPC):
**A61P 1/16; A61K 31/352**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.06.2024 TW 113121391**

(71) Applicant: **Sunway Biotech Co., Ltd.**
**Taipei City 114, (TW)**

(72) Inventors:
- **LEE, Chun-Lin**
  **950 Taitung City, Taitung (TW)**
- **PAN, Tzu-Ming**
  **114 Taipei City (TW)**
- **PAN, Chia Yueh**
  **114 Taipei City (TW)**
- **HSU, Ya-Wen**
  **114 Taipei City (TW)**

(74) Representative: **Becker Kurig & Partner Patentanwälte mbB**
**Bavariastraße 7**
**80336 München (DE)**

Remarks:
The complete document including Reference Table(s) and the Sequence Listing(s) can be downloaded from the EPO website

# (54) COMPOUND FOR PREVENTING OR TREATING NONALCOHOLIC FATTY LIVER DISEASE AND COMPOSITION THEREOF

(57) The present invention provides a compound of Formula (I), which can be used to prevent or treat non-alcoholic fatty liver disease. The present invention also provides a composition comprising an effective amount of the compound, which not only has the efficacy of reducing the accumulation of TC (total cholesterol) and TG (triglycerides) caused by obesity-related nonalcoholic fatty liver disease (NAFLD), but also has the efficacy of reducing serum liver function markers Aspartate aminotransferase (AST) and Alanine aminotransferase (ALT). Therefore, the composition of the present invention has the potential for preventing or treating nonalcoholic fatty liver and related metabolic diseases such as hyperlipidemia.

EP 4 659 748 A1

## Description

## BACKGROUND OF THE INVENTION

### 1. Field of the Invention

The present invention relates to the technical field of preventing or treating nonalcoholic fatty liver disease, and more particularly relates to the technical field involving a compound having the structure of Formula (I) and the composition thereof for preventing or treating nonalcoholic fatty liver disease.

### 2. Description of the Prior Art

Nonalcoholic fatty liver disease (NAFLD) is one of the most common liver diseases in the Western world, affecting approximately 20% to 30% of the general adult population, and the prevalence rate can reach 70% to 90% among obese and diabetic patients. According to surveys and studies conducted by domestic research institutions, the prevalence rate of nonalcoholic fatty liver disease is about 11.5%. It is currently known that insulin resistance is an important cause of nonalcoholic fatty liver disease, hence there is a clear correlation between metabolic syndrome and patients with nonalcoholic fatty liver disease. Increasing evidence suggests that nonalcoholic fatty liver disease increases the risk of cardiovascular diseases, and this risk increases with the severity of nonalcoholic fatty liver disease.

Nonalcoholic fatty liver refers to the excessive accumulation of fat in liver cells, not caused by alcohol. Normally, the liver contains a small amount of fat, but if the weight of fat in the liver exceeds 5%-10%, it is called fatty liver. Nonalcoholic fatty liver often occurs in people who are overweight, obese or have diabetes, high cholesterol, or hypertriglyceridemia. Rapid weight loss and bad dietary habits can also lead to nonalcoholic fatty liver. The harm of nonalcoholic fatty liver to the body should not be underestimated. Over time, fatty liver can lead to liver cirrhosis, and may even result in liver cancer or liver failure. Besides directly causing decompensated cirrhosis, hepatocellular carcinoma, and hepatocellular carcinoma recurrence after liver transplant, nonalcoholic fatty liver can also affect the progression of other chronic liver diseases and participate in the onset of Type 2 diabetes and atherosclerosis. Nonalcoholic fatty liver may cause liver damage similar to that caused by alcohol abuse, and in the most severe cases, it may evolve into liver cirrhosis or liver failure.

Gut microbiota mainly refers to the vast number of bacterial communities residing in the human gut. They have a significant and important impact on various physiological and pathological phenomena of the human body. Fatty liver has a close interaction with the gut microbiota, known as the gut-liver axis. The composition and metabolites of the gut microbiota can affect fat metabolism, inflammatory response, and the degree of fibrosis in the liver. Some studies have found that patients with nonalcoholic fatty liver disease have reduced diversity of gut microbiota, which can have adverse effects due to dysbiosis. Moreover, abnormal gut microbiota can produce harmful substances such as phenylacetic acid, which enter the liver via the hepatic portal vein, promoting the formation and progression of fatty liver. Therefore, adjusting the ecological balance of the gut microbiota, such as increasing the intake of probiotics, may help improve the condition of fatty liver.

Therefore, in order to prevent or treat nonalcoholic fatty liver disease caused by being overweight and obese, the inventors of the present application have devoted considerable effort to research and invention and have finally developed the compound with specific structure in accordance with the present invention and the use thereof. In daily life, patients with obesity and hyperlipidemia can prevent or treat nonalcoholic fatty liver disease through the easily accessible and consumable composition of the present invention without causing adverse effects on the body.

## SUMMARY OF THE INVENTION

The primary objective of the present invention is to provide a compound having the structure of Formula (I), and a composition comprising said compound, wherein the compound and composition can be used for preventing or treating nonalcoholic fatty liver disease and be taken orally.

To achieve the aforementioned objective, the present invention provides a compound having the structure of Formula (I):

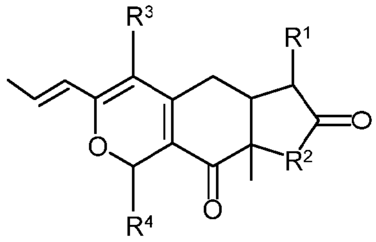

(I)

wherein,

- $R^1$ is alkylcarbonyl or hydroxyalkyl;
- $R^2$ is C or O; and
- $R^3$ and $R^4$ are each independently H, OH, $NH_2$, SH, halogen, CN, $N_3$, $NO_2$, $C_1$-$C_6$ alkyl, $C_1$-$C_6$ alkoxy, or $C_1$-$C_6$ haloalkyl.

Furthermore, in the compound having the structure of Formula (I) of the present invention, the $C_1$-$C_6$ alkyl is straight-chain or branched, saturated or unsaturated, and unsubstituted, monosubstituted or polysubstituted. In addition, the alkylcarbonyl is pentylcarbonyl or heptylcarbonyl, and the hydroxyalkyl is hydroxypentyl.

Moreover, the compound having the structure of Formula (I) of the present invention has a structure selected from Formulae (I-A) to (I-F):

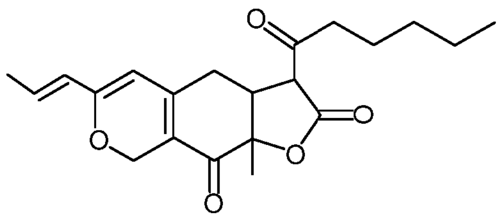

(I-A);

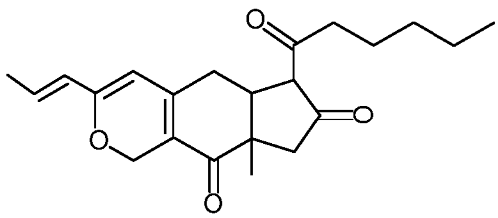

(I-B);

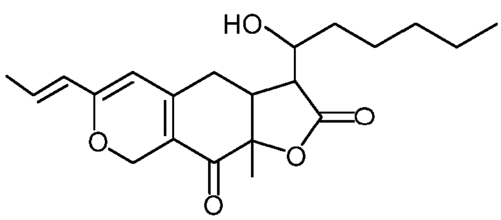

(I-C);

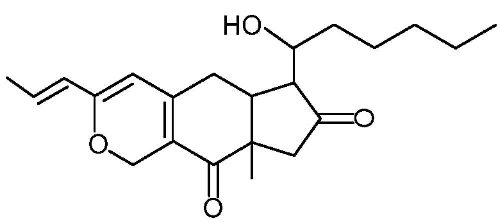

(I-D);

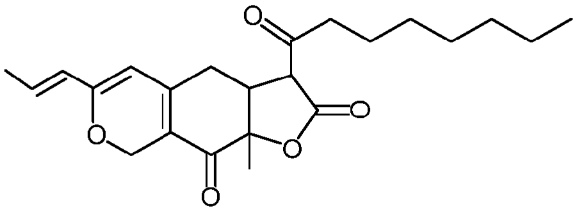

(I-E);

and

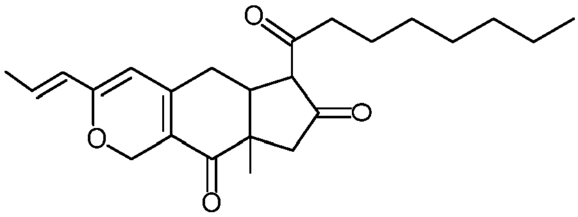

(I-F).

The compound having the structure of Formula (I) of the present invention can be prepared through appropriate chemical synthesis methods, such as condensation, substitution, oxidation-reduction reactions, etc. The raw materials used can be commercially available or prepared according to literature methods.

Furthermore, the present invention provides a composition for preventing or treating nonalcoholic fatty liver disease, characterized by comprising the compound having the structure of Formula (I) as described above.

The nonalcoholic fatty liver disease prevented or treated by the composition of the present invention includes nonalcoholic fatty liver disease caused by at least one condition selected from the group consisting of obesity, hypertension, hyperlipidemia, and diabetes. The composition reduces serum liver function indices AST and ALT, and inhibits the accumulation of fat in liver tissue. Furthermore, the composition reduces the levels of total cholesterol (TC) and triglycerides (TG) in liver, and improves liver lipid droplet accumulation and fatty infiltration caused by nonalcoholic fatty liver disease. Moreover, the composition is capable of enhancing abundance of beneficial intestinal flora and changing gut microbiota composition.

Additionally, an effective amount of the compound in the aforementioned composition of the present invention when used alone is at least 1.5 mg to 12 mg per day for adults.

The aforementioned composition of the present invention may be a food composition, a pharmaceutical composition, a feed composition, a nutritional supplement composition, a dietary supplement composition, or a food additive composition.

As used herein, the term "obesity" is defined as abnormal or excessive fat accumulation that may impair health. Irregular eating habits, excessive food intake, lack of physical activity, endocrine system diseases, genetic factors, psychological factors, and medications can all lead to obesity. Moreover, obesity increases the risk of developing diseases such as atherosclerosis, cardiovascular diseases (stroke and ischemic cardiovascular disease), hypertension, diabetes, hyperlipidemia, and fatty liver.

As used herein, the term "hyperlipidemia" refers to an excess of lipids or fats in the body, such as cholesterol and triglycerides. The term "hypertension" refers to abnormally high blood pressure (the pressure exerted by blood against the walls of blood vessels), and studies have shown that triglycerides are positively correlated with blood pressure (including systolic and diastolic pressure).

As used herein, the term "fatty liver (hepatic steatosis)" is a common condition caused by excessive accumulation of fat in the liver. A healthy liver contains a small amount of fat, but when fat accounts for 5% to 10% of the liver’s weight, it becomes a problem, leading to various diseases such as angina, myocardial infarction, stroke, atherosclerosis, pancreatitis, or similar diseases. Furthermore, fatty liver is divided into alcoholic fatty liver caused by drinking alcohol and nonalcoholic fatty liver disease (NAFLD) not caused by alcohol.

As used herein, the term "nonalcoholic fatty liver" refers to fatty liver caused by excessive accumulation of fat in the liver not due to alcohol, and literature also mentions that obesity, hypertension, hyperlipidemia (such as high cholesterol or triglycerides), and diabetes, among others, may be factors leading to fat accumulation in the liver.

## BRIEF DESCRIPTION OF THE DRAWINGS

FIG. 1 shows a flow chart of the identification of a Monascus pilosus strain of the present invention;
FIG. 2 shows a phylogenetic tree of the Monascus pilosus of the present invention after the alignment of the β-tubulin sequence;
FIG. 3 shows a phylogenetic tree of the Monascus pilosus of the present invention after the alignment of the ITS sequence;
FIG. 4 shows a species-specific PCR analysis result of the Monascus pilosus of the present invention;
FIG. 5 shows a PCR analysis result of pksCT gene of the Monascus pilosus of the present invention;
FIG. 6 shows the morphology of an ascocarp of the Monascus pilosus of the present invention;
FIG. 7 shows the effects of the red yeast rice and monascinol (compound having the structure of Formula (I)) thereof of the present invention on the histopathological sections of the liver of mice fed a long-term high-fat diet;
FIG. 8 shows the effects of the red yeast rice and monascinol (compound having the structure of Formula (I)) thereof of the present invention on the gut microbiota of mice fed a long-term high-fat diet, which displays the relative abundance of the top 10 genera in the gut microbiota as revealed by high-throughput sequencing;
FIG. 9 shows the bacterial abundance of each experimental group (L008, H008, and Msol groups) compared to the HFD group; and
FIG. 10 shows the Partial Least Squares Discriminant Analysis (PLS-DA) of the β-diversity of gut microbiota in each experimental group (ND, HFD, L008, H008, and Msol groups).

## DETAILED DESCRIPTION OF THE PREFERRED EMBODIMENTS

All technical and scientific terms used in the present invention, unless otherwise defined, have the meaning commonly understood by persons of ordinary skill in the art to which the present invention pertains. The present invention is illustrated by the following embodiments, which are illustrative and not limiting, and the present invention is not subject to the limitations of the following embodiments. Unless otherwise noted, the materials used in the present invention are commercially available, and the following are only examples of available sources.

The present invention provides a novel Monascus pilosus, which is deposited at the National Collections of Industrial, Food and Marine Bacteria (NCIMB Ltd) under the Accession Number of NCIMB 44103. In addition, the present invention provides a composition for preventing or treating nonalcoholic fatty liver disease, which contains a Monascus pilosus fermented product with an effective amount, and the Monascus pilosus fermented product is prepared by fermentation using the Monascus pilosus deposited at the National Collections of Industrial, Food and Marine Bacteria (NCIMB Ltd) under the Accession Number of NCIMB 44103. Further, the present invention provides a composition for preventing or treating nonalcoholic fatty liver disease, which contains an active ingredient with an effective amount, and the active ingredient is extracted from a Monascus pilosus fermented product, wherein the Monascus pilosus fermented product is prepared by fermenting a base material by the Monascus pilosus deposited at the National Accession Number of NCIMB 44103. In addition, the active ingredient is Monascinol (compound having the structure of Formula (I)), and the base material is a rice, dioscoreae rhizoma (yam), or mixture of related carbohydrates. Furthermore, the present invention conducts experiments on mice with high-fat diet-induced nonalcoholic fatty liver disease to confirm that the Monascus pilosus and the aforementioned composition prepared therefrom according to the present invention have the function of preventing or treating nonalcoholic fatty liver disease.

Furthermore, the present invention provides a compound having the structure of Formula (I):

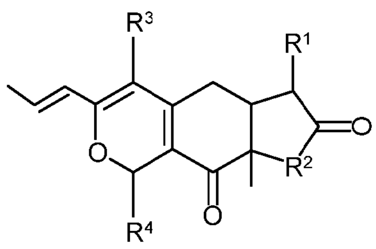

(I)

wherein the compound having the structure of Formula (I), such as Monascinol, can be obtained not only by extraction from Monascus pilosus fermented products but also by appropriate chemical synthesis methods. The synthesis of this compound can be carried out through reactions such as condensation, substitution, and oxidation-reduction, using raw materials that are commercially available or prepared according to literature methods. The compound having the structure of Formula (I) can be used for preparing a composition for preventing or treating nonalcoholic fatty liver disease. The present invention also provides a composition comprising an effective amount of a compound having the structure of Formula (I), which has the ability to prevent or treat nonalcoholic fatty liver disease, wherein the nonalcoholic fatty liver

disease includes nonalcoholic fatty liver disease caused by at least one condition selected from the group consisting of obesity, hypertension, hyperlipidemia, and diabetes. The composition reduces serum liver function indices AST and ALT, and inhibits the accumulation of fat in liver tissue. Furthermore, the composition reduces the levels of total cholesterol (TC) and triglycerides (TG) in liver, and improves liver lipid droplet accumulation and fatty infiltration caused by nonalcoholic fatty liver disease. Moreover, the composition is capable of enhancing abundance of beneficial intestinal flora and changing gut microbiota composition. The compound and composition of the present invention can be applied in food, pharmaceuticals, feed, nutritional supplements, and other applications.

The aforementioned Monascus pilosus strain of the present invention also includes its progeny after subculture, or mutant strains, but still possessing the same strain characteristics, genomic characteristics, or uses (for preventing or treating nonalcoholic fatty liver disease) as those described in the present invention.

The compositions described herein may include, but are not limited to food, beverage, health food, animal drink additive, animal feed additive, animal and human pharmaceutical composition, food additive, beverage additive, etc. that are applicable to the present invention.

The terms "prevent" and "treat" refer to the circumstance that the composition of the present invention is effective to prevent the occurrence of nonalcoholic fatty liver disease or treat nonalcoholic fatty liver disease as compared to a subject who does not use a composition comprising Monascus pilosus of the present invention, or a fermented product thereof, an extract of a fermented product thereof or a compound having the structure of Formula (I).

The term "effective amount" refers to the effective amount that can effectively prevent or treat nonalcoholic fatty liver disease, or can be called "effective amount for treatment" or "effective amount for improvement". The term "pharmaceutically acceptable" refers to substances or compositions that must be compatible with the other components of the prepared substances and not be harmful to patients.

The composition of the present invention can be prepared using techniques well known to those skilled in the art. The aforementioned Monascus pilosus, its fermented product, the extract of its fermented product, or a compound having the structure of Formula (I), along with a pharmaceutically acceptable vehicle, is used for preparing the composition in a dosage form suitable for the present invention, wherein the dosage form includes, but is not limited to solution, emulsion, suspension, powder, tablet, pill, lozenge, troche, capsule or other similar or suitable dosage form for the present invention.

In the aforementioned composition of the present invention, one or more dissolution aids, buffers, preservatives, colorants, spices, flavors, excipients, etc. commonly used in the field of preparations may also be appropriately added as needed.

In another preferred embodiment, the aforementioned composition provided by the present invention is further added into an edible material to prepare a food product or a health product; wherein the edible material includes but is not limited to: water; fluid milk product; milk; concentrated milk; fermented milk, such as yogurt, frozen yogurt, sour milk, lactic fermenting beverage; milk powder; ice cream; cream cheese; dry cheese; soybean milk; fermented soybean milk; fruit and vegetable juice; juice; sports drink; confectionery; jelly; baby food; health food; animal feed; herbal medicine; dietary supplement, etc.

Furthermore, the present invention also provides a method for preventing or treating nonalcoholic fatty liver disease, which comprises administering an effective amount of the aforementioned compound or composition to a subject with excessive liver fat, for preventing or treating nonalcoholic fatty liver disease.

In addition, the present invention also provides a method or use of the aforementioned Monascus pilosus, Monascus pilosus fermented product, the extract of the Monascus pilosus fermented product, or the compound having the structure of Formula (I) in the manufacture of a composition for preventing or treating nonalcoholic fatty liver disease.

The route of administration for the composition for preventing or treating nonalcoholic fatty liver disease provided by the present invention can be appropriately adjusted according to needs and is not particularly limited. A preferred route of administration is oral administration in an appropriate dosage form.

Embodiment 1: Strain identification of a novel Monascus pilosus in accordance with the present invention

1. Basis and Process of the Strain Identification

The traditional classification and identification of Monascus pilosus is mainly based on colony morphology, of which colony size and color are the main bases (Hawksworth and Pitt, 1983). However, some Monascus strains (such as the Monascus pilosus and Monascus ruber) are not easy to classify based on colony appearance. Therefore, molecular subtyping methods are used for the species identification of Monascus. The molecular subtyping method of Monascus is currently based on sequence alignment of β-tubulin and ITS (Park et al., 2004). However, in order to further improve the accuracy of strain identification, the present invention, in addition to the use of sequence alignment of β-tubulin and ITS, also uses the species-specific polymerase chain reaction (PCR) and pksCT gene PCR developed by the inventor's laboratory to further improve the accuracy of strain identification (see FIG. 1).

2. Strain Culture and Deoxyribonucleic Acid (DNA) Extraction

Potato dextrose broth (PDB) medium was purchased from Difco (Becton-Dickinson Diagnostic System, Sparks, MD, USA). For DNA extraction samples, the strains to be tested were cultured in PDB medium at 28°C for 7 days, collected and ground into powder in liquid nitrogen, and dried in a vacuum oven. Then, 0.1 g of dry fungal powder was weighed and put into a 2-mL microcentrifuge tube, and the QIAamp DNA Mini Kit (purchased from QIAGEN N.V, Velno, The Netherlands) was used for DNA extraction.

3. ITS, β-tubulin Sequence Alignment Analysis and Species-Specific PCR Analysis (1) ITS Sequence PCR Amplification

The total volume of the PCR reaction was 25 μL, which contained 1× PCR buffer, 0.05 mM of four kinds of deoxynucleoside triphosphates (dNTPs), 5 U ExSel high fidelity DNA polymerase (purchased from Bertec Enterprise Co., Ltd., Taipei), 0.2 μM of primer ITS 1 (Sequence ID Number 1) and primer ITS4 (Sequence ID Number 2) and 0.16 μg of template DNA. The PCR reaction conditions included an initial denaturation performed at 95°C for 5 min., and then the denaturations at 95°C for 30 sec., at 62°C for 30 sec. and at 70°C for 1 min, which was defined as one cycle, and 35 cycles were carried out. Finally, a denaturation was performed at 70°C for 10 min. before the product was stored at 4°C. The product was confirmed by electrophoresis and then submitted to Genomics BioSci & Tech, Co. Ltd. (Taipei, Taiwan) for DNA sequencing.

(2) β-tubulin Sequence PCR Amplification

The total volume of the PCR reaction was 25 μL, which contained 1× PCR buffer, 0.05 mM of dNTPs, 5 U of Exsel high fidelity DNA polymerase, 0.12 μM of primer β-tubulin F (Sequence ID Number 3) and primer β-tubulin R (Sequence ID Number 4) (Park et al., 2004) and 0.16 μg of template DNA. The PCR reaction conditions included an initial denaturation performed at 95°C for 5 min., and then denaturations at 95°C for 30 second, at 55°C for 2 min., at 70°C for 2 min. which was defined as one cycle, and 35 cycles were carried out. Finally, a denaturation was performed at 70°C for 10 min. before the product was stored at 4°C. The product was confirmed by electrophoresis and then submitted to Genomics BioSci & Tech, Co. Ltd. (Taipei, Taiwan) for DNA sequencing.

(3) Monascus purpureus (M. purpureus) Species-Specific PCR

M. purpureus species-specific PCR was designed based on the residual fragment of the mokH monacolin K biosynthetic gene unique to M. purpureus genome, and capable of effectively identifying whether Monascus belongs to M. purpureus. The total volume of the PCR reaction was 25 μL, which contained 1× PCR buffer, 0.05 mM dNTPs, 5 U of DNA polymerase (SupeTherm GOLD DNA polymerase), 0.12 μM of primer MPuS1 (Sequence ID Number 5) and primer MPuS2 (Sequence ID Number 6) and 0.16 μg of template DNA. The PCR reaction conditions included an initial denaturation performed at 95°C, 10 min., and then denaturations at 95°C for 30 sec. and at 60°C for 1 min. which was defined as one cycle, and 35 cycles were carried out. Finally, a denaturation was performed at 70°C for 10 min. before performing the electrophoresis analysis.

(4) Monascus pilosus/ruber (M. pilosus/ruber) Species-Specific PCR

M. pilosus/ruber species-specific PCR was designed based on the conserved fragment of the FAS gene in the M. ruber genomic pigment biosynthesis gene cluster, and capable of effectively identifying whether Monascus belongs to M. pilosus/ruber. The total volume of the PCR reaction was 25 μL, which contained 1× PCR buffer, 0.05 mM dNTPs, 5 U of SupeTherm GOLD DNA polymerase, 0.12μM of primer RubPil F (Sequence ID Number 7) and primer RubPil R (Sequence ID Number 8) and 0.16 μg of template DNA. The PCR reaction conditions included an initial denaturation performed at 95°C for 10 min., and then denaturations at 95°C for 30 sec., and at 60°C for 1 min. which was defined as one cycle, and 35 cycles were performed. Finally, a denaturation was performed at 70°C for 10 min. before performing the electrophoresis analysis.

(5) pksCT Gene PCR

The pksCT gene is the core gene for the production and synthesis of citrinin in M. purpureus, this gene has been lost in the genome of M. pilosus/ruber, so that this gene can be used as an auxiliary determination basis for the identification of M. pilosus/ruber. The total volume of the PCR reaction was 25 μL, which contained 1× PCR buffer, 0.05 mM of dNTPs, 5 U of SupeTherm GOLD DNA polymerase, 80 nM of primer pksCT-M R (Sequence ID Number 9) and primer pksCT-M F

(Sequence ID Number 10) and 10 ng of template DNA. The PCR reaction conditions included an initial denaturation performed at 95°C for 10 min., and then denaturations performed at 95°C for 30 sec., at 54°C for 30 sec., at 72°C for 40 sec., which was defined as one cycle, and 30 cycles were performed. Finally, a denaturation was carried out at 70°C for 10 min. before performing the electrophoresis analysis.

(6) Sequence Alignment Analysis

Geneious 8.1.9 software (by Biomatters Ltd., Auckland, New Zealand) was used for the sequence alignment analysis. Geneious built-in assembler was used for sequence combination, MAFFT 7.017 was used for multiple alignment, MEGA was used for establishing the phylogenetic tree of maximum likelihood, the nucleic acid substitution mode was General Time Reversible (GTR), and bootstrapping was performed for 1000 times. MrBayes was used for Bayesian phylogenetic tree and post hoc probability test, the nucleic acid substitution mode was GTR, and Aspergillus terreus was used as an out group.

ITS and β-tubulin Sequence Alignment Analysis Results

After the sequences of β-tubulin and ITS of the Monascus pilosus of the present invention were aligned, their phylogenetic trees were as shown in FIGS. 2 and 3 respectively. The sequence alignment result of β-tubulin indicated that the Monascus pilosus of the present invention was of the same branch of M. pilosus and M. ruber, with a bootstrap support of 97% (>50%). The sequence alignment result of ITS indicated that the Monascus pilosus of the present invention was of the same monophyletic group with the branch of M. purpureus, with a bootstrap support of 62% (>50%) (See FIG. 3). The results shown in FIGS. 2 and 3 consistently indicated that the Monascus pilosus of the present invention belonged to either the species of M. pilosus or M. ruber. Species-specific PCR analysis results were consistent with the phylogenetic tree analysis results (See FIG. 4, where RubPil is M. ruber/pilosus-specific, and Mpus is M. purpureus-specific). The pksCT gene PCR results also indicated that the genome of the Monascus pilosus of the present invention did not contain pksCT gene (See FIG. 5, where NTU 568 Monascus purpureus serves as the positive control), belonging to either the species of M. pilosus or M. ruber.

In addition, the current classification for distinguishing M. pilosus and M. ruber resides on whether or not the shell of the ascocarp has pigment (which is usually brown for M. ruber and colorless for M. pilosus). The results indicated that the ascocarps of the Monascus pilosus of the present invention were colorless (See FIG. 6). Therefore, the species of the Monascus pilosus of the present invention was determined to be M. pilosus.

Therefore, in the first embodiment as described above, the results of the ITS sequence alignment, β-tubulin sequence alignment, strain(species)-specific PCR and pksCT gene PCR were all consistent, showing that the Monascus pilosus of the present invention belonged to either the species of M. pilosus or M. ruber. After the morphological observation of ascocarps, the Monascus pilosus of the present invention was determined to be the species of M. pilosus. In addition, we can also know from the above sequence alignment analysis results of ITS and β-tubulin that the Monascus pilosus of the present invention is a novel Monascus pilosus SWM-008 isolate.

Embodiment 2: Culture Method of the Monascus pilosus of the Present Invention

1. Inoculum Culture:

(1) Weigh 2 g of rice flour and place it into a 500-mL triangular culture flask with a linear groove at the bottom. Add 100 mL of reverse osmosis water (RO water), plug a breathable silicone stopper to the opening of the flask and shake well. Sterilize at 1.25 Atmospheric pressure (atm), 121°C for 20 min., and after sterilization, cool the flask at room temperature for later use.

(2) Dig out 3 small pieces of Monascus pilosus inoculum from the petri dish and place them into a 500-mL flask, plug the flask with a breathable silicone stopper, and culture it with shaking in a 30°C incubator at 150-200 rpm for 48 - 72 hours.

2. Solid State Culture

(1) Inoculation: Soak 300 g of rice in RO water overnight, drain the water, wrap it in koji cloth, place it in a koji dish, sterilize (at 121°C for 30 min) and then cool for inoculation. Take out the Monascus pilosus inoculum liquid and inoculate it into the solid base material (10%) and mix thoroughly.

(2) The culture steps are as follows:

a. Culture Conditions: After inoculation, wrap it with koji cloth properly, and put it in a constant temperature and humidity room at a temperature of 30°C and a relative humidity 60% for culture.

b. Water Replenishment: When the Monascus pilosus reproduces rapidly, some of the water in the solid base material will be evaporated due to the rising temperature and most will be consumed by reproduction. Therefore, the base material becomes dry and needs to be replenished with water. Replenish about 30~50 mL of sterile water daily from the 2nd day to the 11th day, and then perform water replenishment every other day until the collection of Monascus pilosus.
c. Collection of Monascus pilosus: Place the Monascus pilosus fermented product that has been cultured for 21 days (culture for 14-28 days) in an oven to dry (at 37°C for 24 hr), and then store it after drying, and this Monascus pilosus fermented product is red yeast rice.

Embodiment 3: Monascinol (a compound having the structure of Formula

(I)) Purification and Separation Method

1. Weigh about 1000 g of red yeast rice.
2. Add about 10 liters (L) of 95% EtOH and perform an extraction in a water bath at 60°C. Shake evenly every 30 minutes, and continue the extraction for 2 hours. Filter the extract with filter paper and repeat the extraction once.
3. Concentrate the filtered extract to a thick form under reduced pressure, add 2 to 2.5 times the weight of silica gel, mix and concentrate them to dryness under reduced pressure, freeze-dry them in a freeze dryer overnight before weighing.
4. Weigh about 7 to 8 times the weight of the silica gel from Step 3 and mix with Hex:EtOAc (8:2) solvent to fill into an open chromatography column. After the chromatography column is balanced, add the mixture of silica gel and extract from Step 3 above. Spread the mixture flatly on top and wait for elution.
5. Wash the chromatography column sequentially with Hex:EtOAc (8:2), Hex: EtOAc (7.5:2.5), Hex:EtOAc (7:3), and Hex:EtOAc (6:4). Collect the Hex: EtOAc (7:3) eluent.
6. Concentrate the Hex:EtOAc (7:3) eluent to dryness under reduced pressure and then redissolve it in MeOH, and finally purify it using preparative HPLC. The solvent conditions are MeOH:dd$H_2O$ (83:17), collect the Monascinol (a compound having the structure of Formula (I)) eluent and concentrate it to dryness under reduced pressure, and finally remove the residual moisture by vacuum drying to obtain pure Monascinol (a compound having the structure of Formula (I)).

Embodiment 4: Method for Conducting Experiments with the Composition of the Present Invention Using an Animal Model of Nonalcoholic Fatty Liver Disease Induced by High-Fat Feed

1. Preparation of the Composition of the Present invention

(1) L008 and H008 groups: The composition is the red yeast rice of the present invention obtained by fermenting rice with the Monascus pilosus of the present invention as described in Embodiment 2. The daily dosage for adults in the L008 and H008 groups is 0.5 g/day and 2 g/day, containing 1.5 mg/day and 6 mg/day of monascinol (a compound having the structure of Formula (I)), respectively.
(2) Msol group: Monascinol (a compound having the structure of Formula (I)), with a daily dosage of 6 mg/day for adults.

2. Experiments on Evaluation of the Effect on Improving Nonalcoholic Fatty Liver Disease

(1) Animal Feeding and Care for Experiments
The experiment used 7-week-old male C57BL/6J strain mice purchased from the National Laboratory Animal Center. The initial body weight was about 23 g, with 12 mice per group. They were housed at 60% relative humidity, room temperature 25 ± 1°C, with a 12-hour light cycle (8:00-20:00). The experiment lasted for 22 weeks, during which food and water were provided ad libitum. Body weight, food intake, and water consumption were measured weekly.
(2) Calculation of Animal Feeding Dosage
The feeding dosage calculation was based on the experiment initial estimation method published by the U.S. Food and Drug Administration in 2005 (Estimating the maximum safe starting dose in initial clinical trials for therapeutics in adult healthy volunteers). Using a 60 kg adult as the standard, when conducting trials on higher experimental animals, the dosage conversion principle is to use 12.3 times the recommended daily intake per kilogram of human body weight (mg/kg bw/day) as the recommended daily intake per kilogram of mouse body weight. The mouse group arrangement and feeding dosage are shown in Table 1. The calculation formula is as follows:

Mouse intake dosage per kilogram of body weight = Human recommended intake ÷ body weight 60 kg × 12.3, from which the daily intake dosage per kilogram of mouse body weight can be calculated.

(3) Grouping and Induction of Experimental Animal

Embodiment 4 uses a high-fat feed to induce a nonalcoholic fatty liver animal model. The animal experiment lasted for 22 weeks. Animal grouping and test substance dosages are shown in Table 1. Animal test feed was prepared according to the formula of Research Diets (New Brunswick, NJ, USA) for D12450B and D12492. The formulations for normal feed and high-fat feed are shown in Table 2. The grouping is as follows:

i. ND group: Normal animal group.
ii. HFD group: Control group, nonalcoholic fatty liver mice induced by high-fat feed.
iii. L008 group: Nonalcoholic fatty liver mice induced by high-fat feed and gavaged daily with red yeast rice powder, equivalent to 0.5 g of the red yeast rice of the present invention consumed daily by a 60 kg adult, containing 1.5 mg monascinol (a compound having the structure of Formula (I)).
iv. H008 group: Nonalcoholic fatty liver mice induced by high-fat feed and gavaged daily with red yeast rice powder, equivalent to 2 g of the red yeast rice of the present invention consumed daily by a 60 kg adult, containing 6 mg monascinol (a compound having the structure of Formula (I)).
v. Msol group: Nonalcoholic fatty liver mice induced by high-fat feed and gavaged daily with monascinol (a compound having the structure of Formula (I)) powder, equivalent to 6 mg of monascinol (a compound having the structure of Formula (I)) powder consumed daily by a 60 kg adult.

Table 1: Animal Grouping and Test Substance Administration for High-Fat Diet-Induced Nonalcoholic Fatty Liver Injury in Mice

| Experimental Group | Feed and Drink | Administered Substance | Adult Dose (g/60 kg bw) | Mouse Dose (g/kg bw/day) |
|---|---|---|---|---|
| ND | Normal Diet | RO Water | 0 | 0 |
| HFD | High Fat Diet | RO Water | 0 | 0 |
| L008 | High Fat Diet | Red yeast rice powder | 0.5 | 0.103 |
| H008 | High Fat Diet | Red yeast rice powder | 2 | 0.410 |
| Msol | High Fat Diet | Monascinol (a compound having the structure of Formula (I)) | 0.006 | 0.0012 |

Table 2: Feed Formulation for High-Fat Diet-Induced Nonalcoholic Mouse Animal Experiment

| | Normal Feed | | High-Fat Feed | |
|---|---|---|---|---|
| (Ingredient) | Content (g) | Calories (kcal) | Content (g) | Calories (kcal) |
| Casein (30 Mesh) | 200 | 800 | 200 | 800 |
| L-cysteine | 3 | 12 | 3 | 12 |
| Corn starch | 315 | 1260 | 0 | 0 |
| Maltodextrin 10 | 35 | 140 | 125 | 500 |
| Sucrose | 350 | 1400 | 68.8 | 275.2 |
| Cellulose, BW200 | 50 | 0 | 50 | 0 |
| Soybean oil | 25 | 225 | 25 | 225 |
| Ghee powder | 20 | 353.5 | 245 | 1732.2 |
| Mineral Mix S10026 | 10 | 0 | 10 | 0 |
| DiCalcium phosphate | 13 | 0 | 13 | 0 |
| Calcium carbonate | 5.5 | 0 | 5.5 | 0 |
| Potassium citrate | 16.5 | 0 | 16.5 | 0 |

(continued)

| (Ingredient) | Normal Feed | | High-Fat Feed | |
|---|---|---|---|---|
| | Content (g) | Calories (kcal) | Content (g) | Calories (kcal) |
| Vitamin Mix V10001 | 10 | 40 | 10 | 40 |
| Choline bitartrate | 2 | 0 | 2 | 0 |

(4) Animal Sacrifice and Blood Collection

In the 22nd week of the experiment, food was withheld for 12-14 hours before blood collection. All mice were euthanized using carbon dioxide, and blood samples were collected via the inferior vena cava. The obtained blood samples were centrifuged at 4°C, 3,000 xg for 15 minutes. The upper layer of blood was aliquoted into eppendorf tubes and stored at -80°C. The liver was weighed, and the second largest lobe was preserved in 10% pathological formalin. The remaining liver was washed with 0.9% physiological saline, and along with the kidneys, placed in zipper bags and stored at -80°C.

(5) Serum Biochemical Analysis

A biochemical auto-analyzer (Model Beckman-700, purchased from Fullerton, CA, USA) was used for detection. The items detected were: TC (Total Cholesterol), TG (Triglyceride), AST (Aspartate Aminotransferase) and ALT (Alanine Aminotransferase) activities.

(6) Liver Lipid Extraction and Measurement

Weigh 0.1 g of liver tissue and add 1 mL of chloroform: methanol (2: 1, v/v). Homogenize with a tissue homogenizer and centrifuge to collect the supernatant. The supernatant was vacuum-dried to remove the solvent, and finally, dimethyl sulfoxide (DMSO) was added to redissolve the sample. The extract was stored at 20°C. Commercial biochemical reagents (Model BXC 0261, Fortress) were used to analyze TC concentration, and commercial biochemical reagents (Model BXC 0271, Fortress) were used to analyze TG concentration, following the instructions in the biochemical reagent kit manual.

(7) Hematoxylin-Eosin (H&E) Staining of Liver Tissue

The liver was fixed in 10% formalin, dehydrated, and embedded in paraffin to make tissue sections. Routine staining was performed with hematoxylin and eosin.

(8) Biostatistical Analysis Method

Experimental results are presented as mean ± standard deviation (mean ± SD). Statistical analysis was performed using one-way ANOVA in the Statistical Package for the Social Sciences (SPSS 12.0) system, followed by Duncan's test for inter-group comparison of differences. $p < 0.05$ indicates a significant difference.

3. Analysis of Gut Microbiota in Nonalcoholic Fatty Liver Mice

(1) Extraction of Genomic DNA and PCR Amplification and Purification

Total genomic DNA was extracted from samples (QIAamp PowerFecal DNA, Qiagen). DNA concentration was determined using a Qubit 4.0 fluorometer (Thermo Scientific) and adjusted to 1 ng/μL. The full-length 16S gene (V1-V9 region) was amplified using barcoded 16S gene-specific primers (Forward primer 1: 5'Phos/GCATC-16-base barcode - Sequence ID Number 11, Reverse primer 2: 5'Phos/GCATC- 16-base barcode - Sequence ID Number 12). PCR reactions were performed using KAPA HiFi HotStart ReadyMix (Roche): 95°C, 3 minutes; 20-27 cycles (sample-dependent): 95°C, 30 seconds, 57°C, 30 seconds, 72°C, 60 seconds; 72°C for 5 minutes, and final storage at 4°C. PCR products were monitored on 1% agarose gel. Samples with bright main bands of about 1500 bp were selected using AMPure PB Beads and purified for SMRTbell library preparation.

(2) SMRTbell Library Construction and Sequencing

SMRTbell libraries were prepared according to the procedure for multiplex SMRTbell library preparation and sequencing (PacBio) using barcoded primers for full-length 16S gene amplification. Sequencing was performed on a PacBio Sequel IIe instrument in circular consensus sequencing (CCS) mode, generating HiFi reads with predicted accuracy (Phred Scale) = 30. The DNA to be tested will be sequenced repeatedly. By comparing the results of repeated sequencing, sequencing errors will be corrected, ultimately achieving high accuracy of >99.9% (QV30).

(3) Bioinformatics Analysis

i. Sequencing Data Processing

Sample data were demultiplexed from the raw data based on Barcode sequences and PCR amplification primer sequences. After removing Barcode and primer sequences, the data were imported into QIIME2 (v2019.7.0; https://qiime2.org/) (Bolyen et al., 2019). Sequences from all samples were stored as a corresponding artifact. QIIME2 cutadapt was used to remove amplicon variant region primers (forward primer 2: Sequence ID Number 13; reverse primer 2: Sequence ID Number 14). The sequences with primers removed were used as QIIME2 DADA2 Input Reads.

ii. QIIME2 DADA2 Denoising and Species Annotation

QIIME2 DADA2 (v2019.7.0; https://qiime2.org/) (Callahan et al., 2016; Quin et al., 2018) denoising analysis first involved sequence filtering, setting each sequence to be trimmed to a specified length (forward reads: 280 bp; reverse reads: 220 bp) and setting MaxEE parameters (forward reads: 2; reverse reads: 2) as the maximum number of expected error bases allowed per sequence. Next, the DADA2 core algorithm denoising was performed, using information such as sequence abundance, quality scores, and relationships between sequences to correct sequencing errors and infer true sequences. After denoising was completed for both ends of the sequences, merging was performed (minimum overlap length 20 bp; no mismatches allowed in the overlap region). Finally, chimeras were removed from the merged sequences by comparing them with higher abundance sequences in the sample. If a sequence had low abundance and was similar to multiple sequences, it was determined to be a chimera and removed. The sequences after denoising analysis were used as representative sequences for Amplicon Sequence Variants (ASVs), and anASV table was obtained for subsequent species annotation. QIIME2 feature-classifier (v2019.7.0; https://qiime2.org/) (Bokulich et al., 2018) was used to select the machine learning-based classification method classify-sklearn (McKinney, 2010; Pedregosa et al., 2011) and train naive Bayes classifiers (Wang et al., 2007) for different databases and specific variant regions. This classifier was used for ASV representative sequence species classification and annotation, obtaining classification information and statistics on microbial composition for each sample at various taxonomic levels: kingdom, phylum, class, order, family, genus, and species. 16S analysis used the QIIME2 alignment MAFFT method (v2019.7.0; https://qiime2.org/) (Katoh & Standley, 2013; Lane, 1991) with the GreenGenes database (gg_13_8) (DeSantis et al., 2006; McDonald et al., 2012) or Silva database (v132; 2017.12) (Quast et al., 2012) Core Set (representing most major prokaryotic taxonomic units) for rapid sequence alignment. The latest NCBI database could also be used for sequence alignment to obtain annotation information (Balvočiūtė & Huson, 2017; Gyarmati et al., 2016; Hong et al., 2016). ITS analysis used the unite database (v7.2; 2017.12.01) (Abarenkov et al., 2010; Kõljalg et al., 2005; Kõljalg et al., 2013; Nilsson et al., 2019) for annotation. Finally, sequence data for each sample were uniformized, using the smallest total number of tags in the samples as the standard for uniformization (Schloss et al., 2009). The processed data were then used for subsequent Beta diversity analysis.

iii. Sample Group Comparison Analysis (Beta diversity)

Qiime was used to calculate UniFrac distances and construct UPGMA sample clustering trees. R (v3.3.1) was used to plot PLS-DA analysis graphs. PLS-DA analysis used R's mixOmics and ggplot2 packages, and Ternary Plot analysis used R's ggtern package. R's agricolae package was used for Beta diversity index inter-group difference analysis, performing both parametric and non-parametric tests, using Tukey's test and Kruskal's test (post-hoc test).
The experiment was conducted according to the experimental method described in Embodiment 4, and the results are as follows:

1. The Effect of the Composition of the Present Invention on Liver Weight and Liver/Body Weight Ratio in Mice with Nonalcoholic Fatty Liver Disease

The results in Table 3 indicate that, compared with the ND group, the HFD group resulted in an abnormal increase

in liver weight. This phenomenon may suggest that a long-term high-fat diet leads to abnormal liver tissue due to fat accumulation. However, this condition can be significantly improved by feeding mice various doses of the Monascus pilosus fermented product (L008 and H008 groups) and Msol (a compound having the structure of Formula (I)) from the present invention, significantly reducing liver weight ($p<0.05$), and also improving the liver/body weight ratio.

Table 3 The Effect of Red yeast Rice and Monascinol (a compound having the structure of Formula (I)) thereof from the Present Invention on Liver Weight and Liver/Body Weight Ratio in Mice fed a Long-Term High-Fat Diet

| Experimental Group | Liver Weight (g) | Liver/Body Weight (%) |
|---|---|---|
| ND | 1.04 ± 0.16 [a] | 3.04 ± 0.30 [a] |
| HFD | 1.77 ± 0.35 [b] | 3.89 ± 0.58 [b] |
| L008 | 1.06 ± 0.06[a] | 3.06 ± 0.16 [a] |
| H008 | 1.03 ± 0.06 [a] | 3.04 ± 0.09 [a] |
| Msol | 1.01 ± 0.06 [a] | 2.85 ± 0.24 [a] |

The experimental data are represented as mean ± standard deviation (mean ± SD) (n=8). Mean values with different letters indicate significant differences ($p<0.05$). The statistical significance of biochemical effects was determined using one-way analysis of variance (ANOVA) and Duncan's multiple test.

2. The Effect of the Composition of the Present Invention on Serum Liver Function Indices AST and ALT in Mice with Nonalcoholic Fatty Liver Disease

As observed in Table 4, the serum AST and ALT activities in the HFD group were significantly higher than those in the ND group ($p<0.05$). This result indicates that a long-term high-fat diet may cause liver cell damage, leading to liver injury associated with nonalcoholic fatty liver disease. Feeding various doses of the Monascus pilosus fermented product (L008 and H008 groups) and Monascinol of the present invention significantly reduced AST and ALT activities induced by a long-term high-fat diet. The low-dose group (L008 group) of red yeast rice of the present invention, equivalent to a daily intake of 0.5 g red yeast rice for adults, showed a significant reduction in AST and ALT activities. In addition, the intake of Monascinol (a compound having the structure of Formula (I)), equivalent to a daily adult consumption of 6 mg, also improved AST and ALT levels.

Table 4 The Effect of the Red Yeast Rice and Monascinol (a compound having the structure of Formula (I)) from the Present Invention on Serum Liver Function Indices AST and ALT in Mice Fed a Long-Term High-Fat Diet

| Experimental Group | AST Activity (U/L) | ALT Activity (U/L) |
|---|---|---|
| ND | 59.9 ± 4.6 [a] | 22.8 ± 3.1[a] |
| HFD | 158.1 ± 53.8 [b] | 136.5 ± 82.1 [b] |
| L008 | 70.4 ± 8.3 [a] | 34.3 ± 11.1 [a] |
| H008 | 59.0 ± 6.7 [a] | 29.3 ± 5.1 [a] |
| Msol | 60.6 ± 3.7[a] | 25.3 ± 3.4 [a] |

The experimental data are represented as mean ± standard deviation (mean ± SD) (n=8). Mean values with different letters indicate significant differences ($p<0.05$). The statistical significance of biochemical effects was determined using one-way analysis of variance (ANOVA) and Duncan's multiple test.

3. The Effect of the Composition of the Present Invention on Liver TG and TC Levels in Mice with Nonalcoholic Fatty Liver Disease

As shown in Table 5, the lipid analysis of the liver indicated that the HFD group increased the TC and TG concentrations in the liver ($p<0.05$). The low-dose group (L008 group) of red yeast rice of the present invention exhibited a slight reduction in TG production in the liver induced by a long-term high-fat diet and significantly reduced TC levels. Feeding red yeast rice powder, even without reaching a high dose (H008 group), can already significantly reduce both TC and TG levels in the liver ($p<0.05$). The pure substance Msol group also effectively reduced the accumulation of TC and TG in the liver ($p<0.05$). These results demonstrate that the red yeast rice and Monascinol from the present invention have the effect of reducing the accumulation of TC and TG in the liver, showing potential for improving fatty liver. This result also confirms that Monascinol (a compound having the structure of Formula (I)) is the active ingredient in red yeast rice

responsible for reducing liver lipid levels.

Table 5 The Effect of Red Yeast Rice and Monascinol (a compound having the structure of Formula (I)) of the Present Invention on Liver TG and TC Levels in Mice fed a Long-Term High-Fat Diet

| Experimental Group | Liver TG (mg/g) | Liver TC (mg/g) |
|---|---|---|
| ND | 7.36 ± 0.83 [a] | 0.67 ± 0.12 [a] |
| HFD | 8.33 ± 0.91 [b] | 1.67 ± 0.12 [c] |
| L008 | 7.88 ± 1.02 [ab] | 1.16 ± 0.12[b] |
| H008 | 7.12 ± 0.42 [a] | 1.01 ± 0.14 [ab] |
| Msol | 7.25 ± 0.71 [a] | 1.02 ± 0.32 [ab] |

The experimental data are represented as mean ± standard deviation (mean ± SD) (n=8). Mean values with different letters indicate significant differences ($p<0.05$). The statistical significance of biochemical effects was determined using one-way analysis of variance (ANOVA) and Duncan's multiple test.

4. The Effect of the Composition of the Present Invention on Liver Histopathological Sections of Nonalcoholic Fatty Liver Mice

FIG 7 shows the histopathological sections of liver tissue from experimental animals. The results indicate that long-term consumption of a high-fat diet causes the liver tissue of the HFD group to exhibit a large number of lipid droplets, presenting a state of fatty infiltration. As shown in the liver section pathological assessment in Table 6, feeding the red yeast rice and its Monascinol (a compound having the structure of Formula (I)) of the present invention can improve the accumulation of lipid droplets ($p<0.05$), with the H008 group showing better improvement than the L008 group, demonstrating a dose-dependent effect. The Msol (a compound having the structure of Formula (I)) group also significantly improved the accumulation of liver lipid droplets and fatty infiltration in nonalcoholic fatty liver mice induced by a high-fat diet, reducing the occurrence of fatty oxidative lesions.

Table 6. The Effect of Red Yeast Rice and Monascinol (a compound having the structure of Formula (I)) of the Present Invention on Liver Pathological Assessment of Nonalcoholic Fatty Liver Mice

| Experimental Group | Fatty change, general | Fatty change with micro-vesicles, multifocal | Fatty change with macro-vesicles, multifocal | Inflammation, granulomatous, multifocal |
|---|---|---|---|---|
| ND | 0[a] | 0[a] | 0[a] | 0[a] |
| HFD | 2.9 ± 1.4[b] | 2.8 ± 1.3[b] | 1.0 ± 0.5[b] | 0.1 ± 0.4[a] |
| L008 | 0.6 ± 0.9[a] | 0.4 ± 0.5[a] | 0.6 ± 0.9[ab] | 0 [a] |
| H008 | 0.3 ± 0.7[a] | 0.3 ± 0.7[a] | 0.1 ± 0.4[a] | 0 [a] |
| Msol | 0.3 ± 0.7[a] | 0.1 ± 0.4[a] | 0.3 ± 0.7[a] | 0[a] |

Lesions are graded from 1 to 5 based on severity: 0 = no significant lesions; 1 = mild (<10%); 2 = moderate (10-33%); 3 = moderate/heavy (33-66%); 4 = severe/high (66-100%). Data are presented as mean ± standard deviation (n = 8). Means with different letters are significantly different ($p<0.05$). Statistical significance of biochemical effects was determined by analysis of variance (ANOVA) and Duncan's multiple comparison test. Experimental data are presented as mean ± standard deviation (mean ± SD) (n=8). Means with different letters indicate significant differences ($p<0.05$). Statistical significance of biochemical effects was determined using one-way analysis of variance (ANOVA) and Duncan's multiple test.

5. The Effect of Feeding the Red Yeast Rice and Its Indicator Active Ingredient of the Present Invention on the Gut Microbiota of Nonalcoholic Fatty Liver Mice

The results of bacterial abundance analysis are shown in FIG. 8. After induction by a high-fat diet, compared with the normal group, there were significant changes in the types and abundance of bacterial species, especially in the Lactobacillus genus, which showed a significant decrease. At the genus level, the top 10 genera with changes in bacterial abundance were Lachnospiraceae, Dubosiella, Lactobacillus, Bacteroides, [Eubacterium]_coprostanoligen, Ruminococcaceae, Ruminiclostridium, Blautia, Akkermansia, and Parabacteroides.

As shown in FIG 9, compared with the HFD group, feeding the red yeast rice (L008 and H008 groups) of the present invention and Monascinol (a compound having the structure of Formula (I)) (Msol group) can increase the microbial populations of Blautia and Intestinimonas in the intestine. Blautia is one of the most abundant genera in the intestinal microbiota and plays a regulatory role in inflammatory responses and metabolic diseases, being a common probiotic genus. Intestinimonas is characterized by high butyrate production, which has anti-inflammatory and anti-obesity effects (Cai et al., 2020). In this study, all experimental groups showed increased abundance of Blautia and Intestinimonas compared to the high-fat diet group.

Partial least squares discriminant analysis (PLS-DA) was used to analyze the bacterial community structure between different groups. As shown in FIG. 10, the bacterial community distribution of nonalcoholic fatty liver mice after a high-fat diet was significantly different from that of the normal group. Further analysis was conducted to determine whether the composition (test samples) of the present invention could effectively improve the bacterial colony structure of the high-fat diet. The results showed that the red yeast rice L008 and H008 groups of the present invention had similar bacterial colony structures and were significantly separated from the HFD group. In the analysis of bacterial community structure, the Msol group of the active ingredient (Monascinol, a compound having the structure of Formula (I)) of the red yeast rice of the present invention, although similar to the HFD group, showed significant differences in the analysis of bacterial community abundance.

Nonalcoholic fatty liver disease (NAFLD) is characterized by excessive accumulation of fat in the liver causing degeneration, leading to symptoms such as nonalcoholic steatohepatitis (NASH). In the animal experiment of Embodiment 4 of the present invention, it has been demonstrated that the red yeast rice and its active ingredient (Monascinol, a compound having the structure of Formula (I)) of the present invention can improve the ratio of TC and TG in the liver of nonalcoholic fatty liver mice ($p<0.05$), reduce the liver weight/body weight ratio ($p<0.05$), and the liver section and interpretation also confirmed that each test group can significantly alleviate the symptoms of liver fat accumulation, fat vacuoles, and fatty infiltration caused by long-term high-fat diet, proving that the red yeast rice and Monascinol (a compound having the structure of Formula (I)) of the present invention have the effect of improving NAFLD.

Moreover, the ecology of gut microbiota is highly correlated with host health or disease, including changes in the composition ratio of gut microbiota and microbial metabolites (Jiang et al., 2020). Compared to the high-fat diet group (HFD group), the red yeast rice (L008 and H008 groups) and Monascinol (a compound having the structure of Formula (I)) (Msol group) in the composition (test substance) of the present invention can increase the abundance of Blautia genus. Literature indicates that Blautia content is highly negatively correlated with visceral fat accumulation, and its metabolic mechanism can effectively reduce visceral fat (Ozato et al., 2019). Other literature states that Blautia producta, a commensal microorganism in the Blautia genus in the intestine, produces short-chain fatty acids such as acetate, which improves insulin sensitivity in the liver and prevents the occurrence of NAFLD through the acetate-FFAR2 signaling pathway (Aoki et al., 2021). The Intestinimonas genus is characterized by high butyrate production. Previous studies have confirmed that Intestinimonas can alleviate the progression of NAFLD through butyrate and has the potential for antioxidant and anti-inflammatory effects (Miao et al., 2022; Wang et al., 2021). The red yeast rice and Monascinol (a compound having the structure of Formula (I)) of the present invention can also increase the abundance of Intestinimonas, which helps to improve and slow down the progression of NAFLD.

The experimental results of Embodiment 4 of the present invention show that long-term high-fat diet causes nonalcoholic fatty liver in normal mice and affects the composition ratio of their gut microbiota. The red yeast rice and Monascinol (a compound having the structure of Formula (I)) of the present invention can improve specific gut microbiota, such as Blautia and Intestinimonas, thereby regulating liver lipid metabolism, slowing down the progression of NAFLD, and having the effect of improving NAFLD.

Furthermore, the present invention evaluates the improvement effect on fatty liver in mice induced by a 22-week high-fat diet using different test substances (compositions) in different groups (L008 group, H008 group, and Msol group). The following conclusions are summarized from the above embodiments and results:

1. The red yeast rice (L008 and H008 groups) of the present invention contains Monascinol (a compound having the structure of Formula (I)), which has been less frequently proposed to have the effect of reducing nonalcoholic fatty liver. The red yeast rice of the present invention shows significant effects in improving liver weight and liver/body weight ratio, serum liver function indices AST and ALT activities, TG and TC content in the liver, and fatty infiltration in liver tissue. The low dose of red yeast rice powder (L008 group) at 0.5 g per person per day and Monascinol (a compound having the structure of Formula (I)) at 6 mg per person per day are relatively low doses that can prevent and improve fatty liver, demonstrating considerable progressiveness. The present invention also shows that Monascinol (a compound having the structure of Formula (I)) is a new active ingredient for preventing and improving fatty liver.
2. Both the red yeast rice (L008 and H008 groups) and Monascinol (a compound having the structure of Formula (I)) (Msol group) of the present invention can improve the changes in gut microbiota composition caused by a high-fat diet. Compared to the HFD group, they can increase the abundance of Blautia and Intestinimonas genera in the intestine. These genera have the potential to increase short-chain fatty acid metabolism, antioxidant and anti-inflammatory

effects, and improve NAFLD. This confirms that the mechanism of action of the red yeast rice and Monascinol (a compound having the structure of Formula (I)) of the present invention in improving NAFLD is partly highly correlated with enhancing changes in specific intestinal flora.

Embodiment 5: Compound Having the Structure of Formula (I) for Preventing or Treating Nonalcoholic Fatty Liver Disease

The Monascus pilosus fermented product of the present invention contains multiple active ingredients, such as functional components having the structure of Formula (I) including Monascinol, Ankaflavin and Monascin. These components have various physiological activities that can prevent or treat nonalcoholic fatty liver disease. Therefore, the present invention also provides a compound having the structure of Formula (I) as follows:

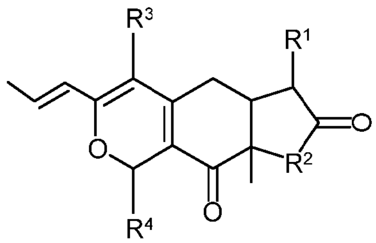

(I)

wherein,

$R^1$ is alkylcarbonyl or hydroxyalkyl;
$R^2$ is C or O; and
$R^3$ and $R^4$ are each independently H, OH, $NH_2$, SH, halogen, CN, $N_3$, $NO_2$, $C_1$-$C_6$ alkyl, $C_1$-$C_6$ alkoxy, or $C_1$-$C_6$ haloalkyl;
wherein the $C_1$-$C_6$ alkyl is straight-chain or branched, saturated or unsaturated, and unsubstituted, monosubstituted or polysubstituted;
wherein the alkylcarbonyl may be pentylcarbonyl or heptylcarbonyl.

Furthermore, the compound having the structure of Formula (I) has a structure selected from any one of Formulae (I-A) to (I-F):

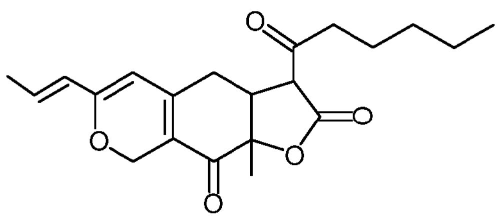

(I-A);

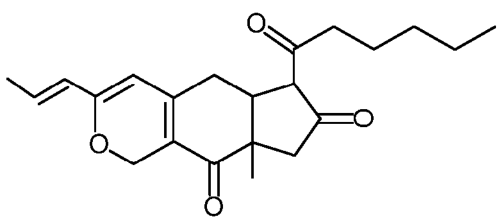

(I-B);

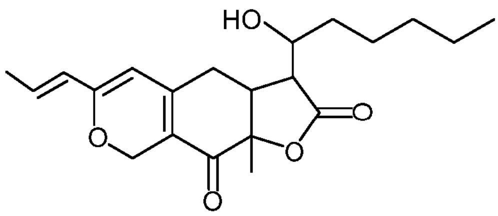

(I-C);

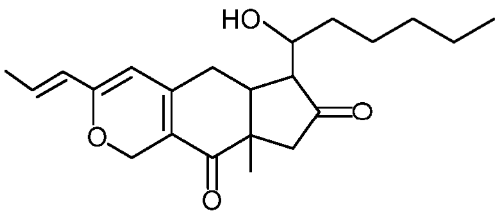

(I-D);

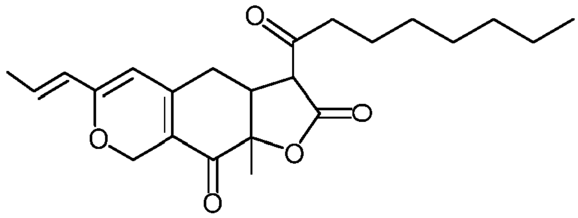

(I-E);

and

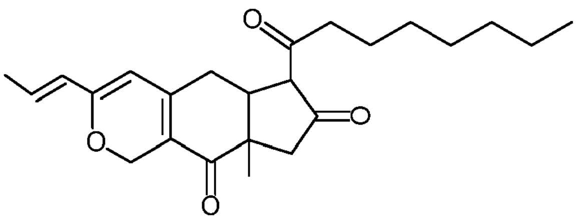

(I-F).

In addition, the compound having the structure of Formula (I) can be obtained by extraction from Monascus pilosus fermented product as described in Embodiment 3 above, such as active ingredients having the structure of Formula (I) including Monascinol, Ankaflavin and Monascin, and can also be prepared by appropriate chemical synthesis methods. The synthesis of this compound can be carried out by reactions such as condensation, substitution, and oxidation-reduction, using raw materials that are commercially available or prepared according to literature methods. The compound having the structure of Formula (I) can be used to prepare a composition for preventing or treating nonalcoholic fatty liver disease.

The compound having the structure of Formula (I) and the composition comprising it provided by the present invention can be used for preventing or treating nonalcoholic fatty liver disease, wherein the nonalcoholic fatty liver disease is caused by at least one condition selected from the group consisting of obesity, hypertension, hyperlipidemia, and diabetes. The composition reduces serum liver function indices AST and ALT, and inhibits the accumulation of fat in liver tissue. Furthermore, the composition reduces the levels of total cholesterol (TC) and triglycerides (TG) in liver, and improves liver lipid droplet accumulation and fatty infiltration caused by nonalcoholic fatty liver disease. The effective amount of the compound in the composition of the present invention when used alone is at least 3 mg to 12 mg per day for adults. In addition, the composition is capable of enhancing abundance of beneficial intestinal flora and changing gut microbiota composition. The compound and composition of the present invention can be applied in food, pharmaceuticals, feed, nutritional supplements, and other uses.

The description above has completely and clearly described the novel Monascus pilosus of the present invention, the compound having the structure of Formula (I), its composition, and its use for preventing or treating nonalcoholic fatty liver disease. It must be emphasized that the above detailed description is a specific description of feasible embodiments of the present invention, but these embodiments are not intended to limit the scope of the patent of the present invention.

Any equivalent implementation or modification made without departing from the technical spirit of the present invention should be included in the scope of the patent claims of the present invention.

Deposit of Microorganisms

The Monascus pilosus of the present invention was deposited at the National Collections of Industrial, Food and Marine Bacteria (NCIMB Ltd) in the United Kingdom under the Accession Number of NCIMB 44103 on January 12, 2023.

## Claims

1. A compound for preventing or treating nonalcoholic fatty liver disease, the compound having the structure of Formula (I):

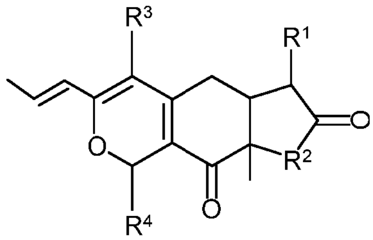

(I);

wherein,

$R^1$ is alkylcarbonyl or hydroxyalkyl;
$R^2$ is C or O; and
$R^3$ and $R^4$ are each independently H, OH, $NH_2$, SH, halogen, CN, $N_3$, $NO_2$, $C_1$-$C_6$ alkyl, $C_1$-$C_6$ alkoxy, or $C_1$-$C_6$ haloalkyl.

2. The compound according to claim 1, wherein the $C_1$-$C_6$ alkyl is straight-chain or branched, saturated or unsaturated, and unsubstituted, monosubstituted or polysubstituted.

3. The compound according to claim 1, wherein the alkylcarbonyl is pentylcarbonyl or heptylcarbonyl.

4. The compound according to claim 1, wherein the hydroxyalkyl is hydroxypentyl.

5. The compound according to claim 1, having a structure selected from Formulae (I-A) to (I-F):

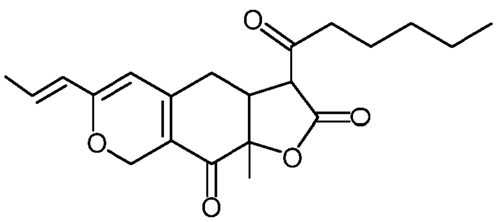

(I-A);

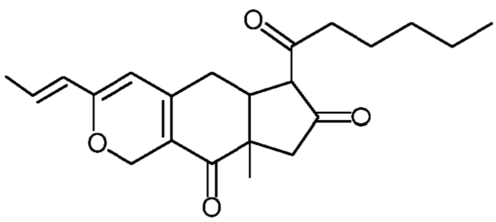

(I-B);

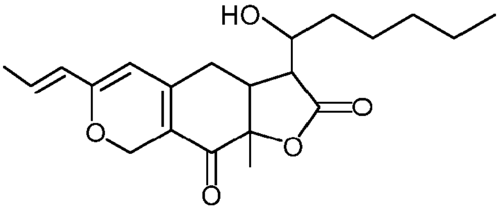

(I-C);

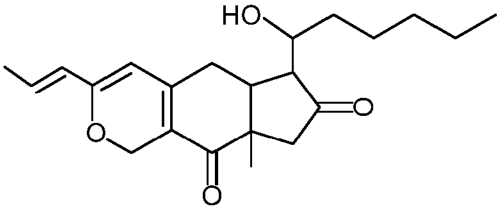

(I-D);

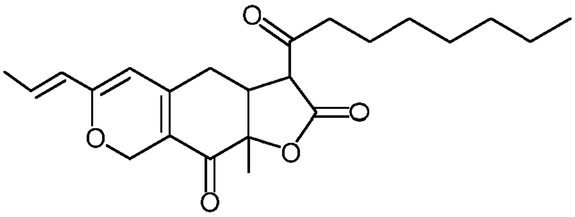

(I-E);

and

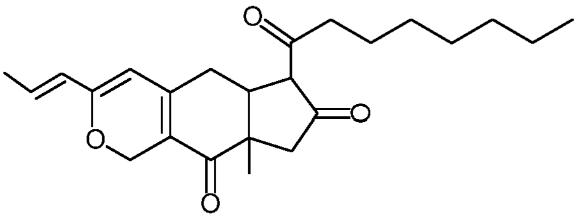

(I-F).

6. A composition for preventing or treating nonalcoholic fatty liver disease, **characterized by** comprising the compound according to claim 1.

7. The composition according to claim 6, wherein the nonalcoholic fatty liver disease is caused by at least one condition selected from the group consisting of obesity, hypertension, hyperlipidemia, and diabetes.

8. The composition according to claim 6, wherein the composition reduces serum liver function indices AST and ALT.

9. The composition according to claim 6, wherein the composition inhibits the accumulation of fat in liver tissue.

10. The composition according to claim 6, wherein the composition reduces the levels of total cholesterol (TC) and triglycerides (TG) in liver.

11. The composition according to claim 6, wherein the composition improves liver lipid droplet accumulation and fatty infiltration.

12. The composition according to claim 6, wherein the composition is capable of enhancing abundance of beneficial intestinal flora and changing gut microbiota composition.

13. The composition according to claim 6, wherein an effective amount of the compound in the composition when used alone is at least 1.5 mg to 12 mg per day for adults.

**14.** The composition according to claim 6, wherein the composition is a food composition, a pharmaceutical composition, a feed composition, a nutritional supplement composition, a dietary supplement composition, or a food additive composition.

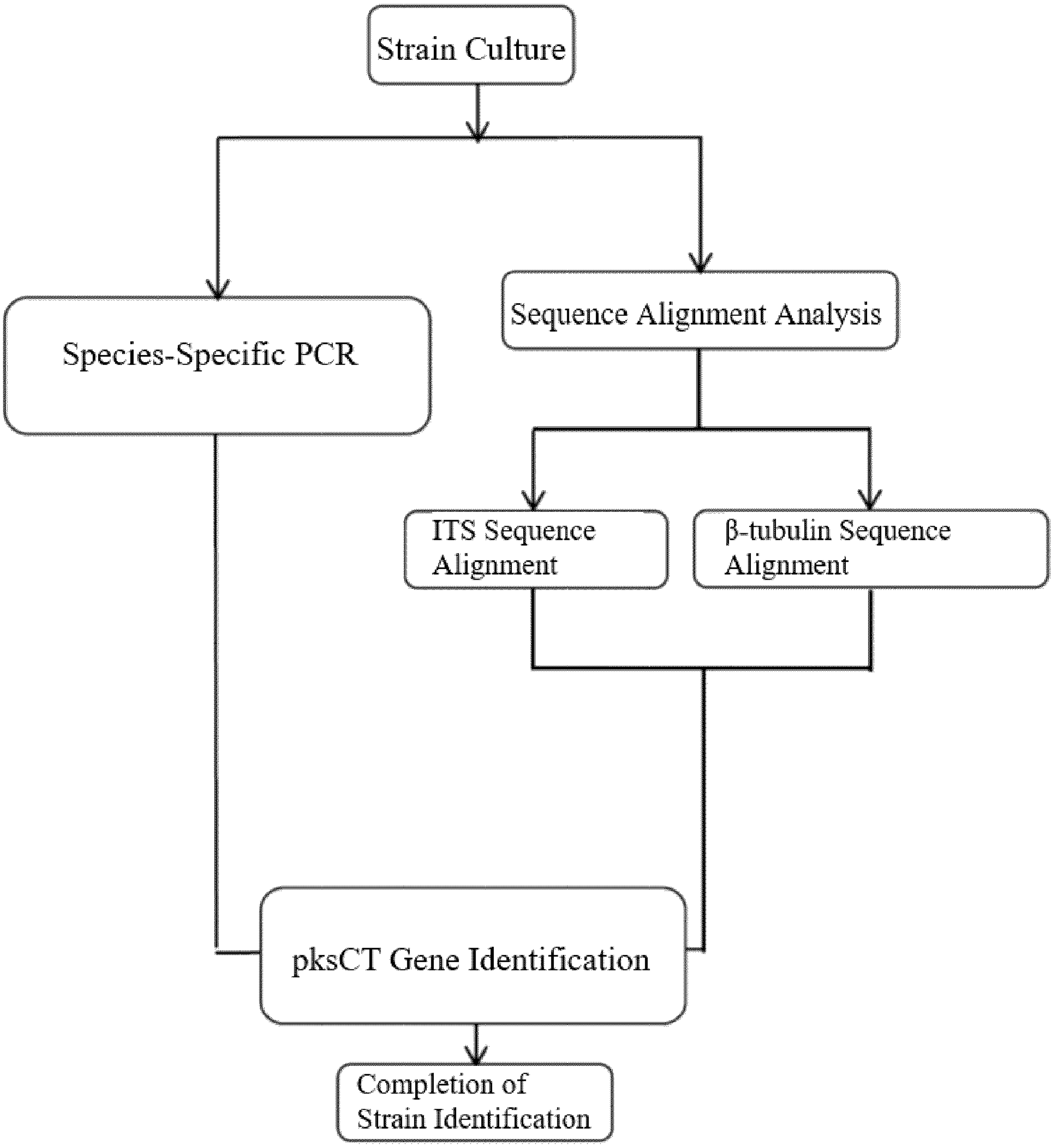
Strain Culture
Species-Specific PCR
Sequence Alignment Analysis
ITS Sequence Alignment
β-tubulin Sequence Alignment
pksCT Gene Identification
Completion of Strain Identification

FIG. 1

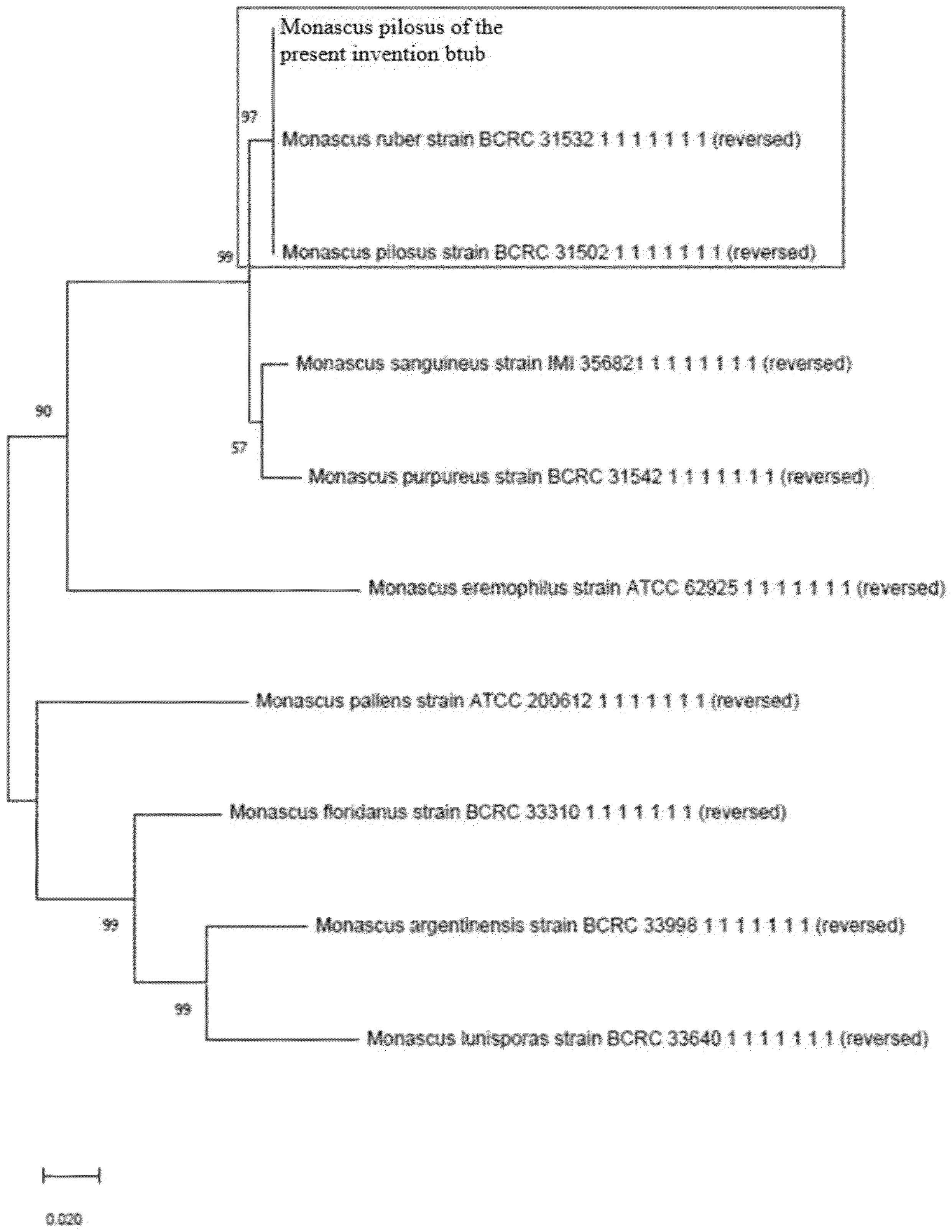
Monascus pilosus of the present invention btub
97
Monascus ruber strain BCRC 31532 1 1 1 1 1 1 1 (reversed)
99
Monascus pilosus strain BCRC 31502 1 1 1 1 1 1 1 (reversed)
Monascus sanguineus strain IMI 356821 1 1 1 1 1 1 1 (reversed)
90
57
Monascus purpureus strain BCRC 31542 1 1 1 1 1 1 1 (reversed)
Monascus eremophilus strain ATCC 62925 1 1 1 1 1 1 1 (reversed)
Monascus pallens strain ATCC 200612 1 1 1 1 1 1 1 (reversed)
Monascus floridanus strain BCRC 33310 1 1 1 1 1 1 1 (reversed)
99
Monascus argentinensis strain BCRC 33998 1 1 1 1 1 1 1 (reversed)
99
Monascus lunisporas strain BCRC 33640 1 1 1 1 1 1 1 (reversed)
0.020

FIG. 2

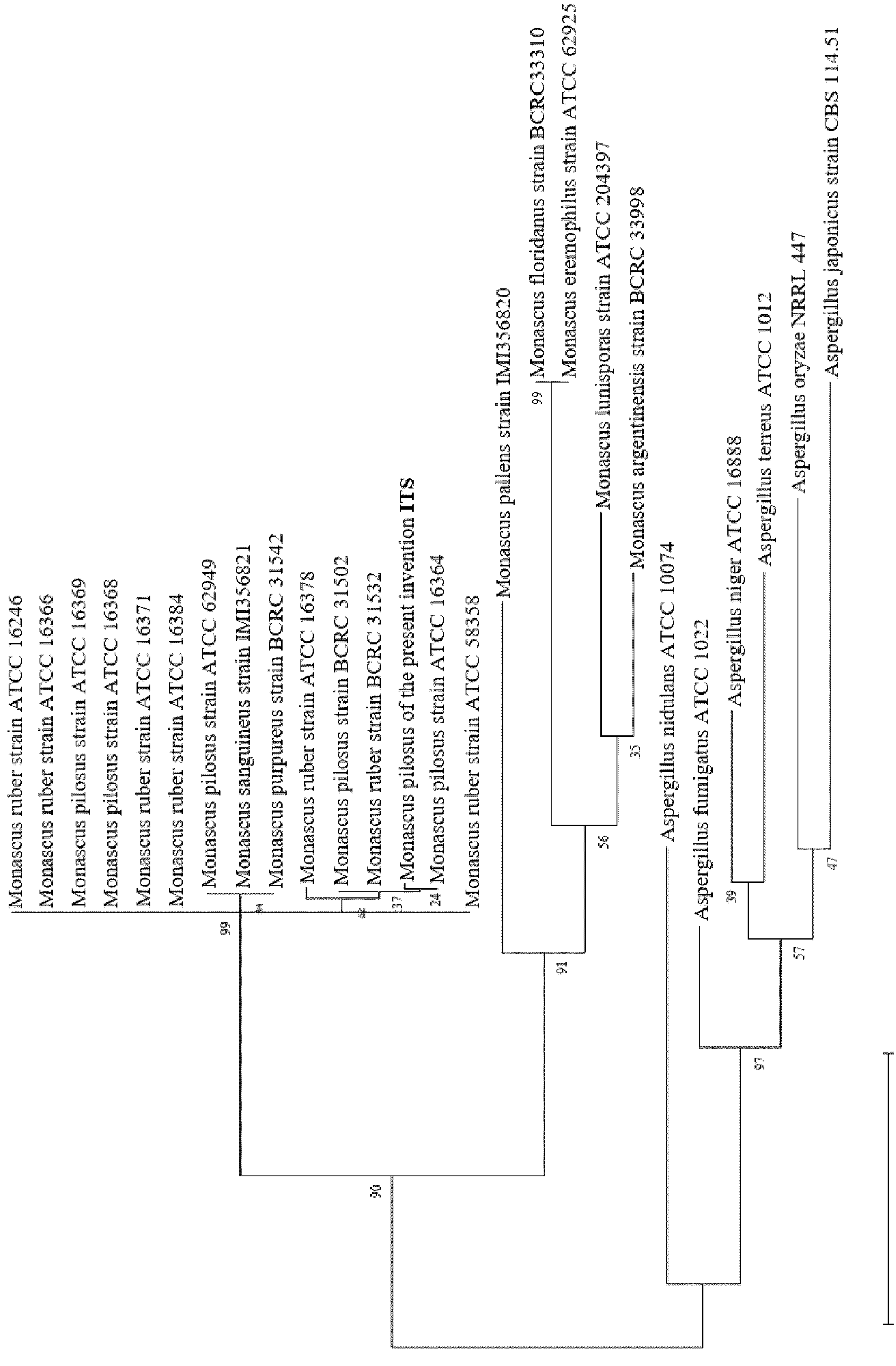

Monascus ruber strain ATCC 16246
Monascus ruber strain ATCC 16366
Monascus pilosus strain ATCC 16369
Monascus pilosus strain ATCC 16368
Monascus ruber strain ATCC 16371
Monascus ruber strain ATCC 16384
Monascus pilosus strain ATCC 62949
Monascus sanguineus strain IMI356821
Monascus purpureus strain BCRC 31542
Monascus ruber strain ATCC 16378
Monascus pilosus strain BCRC 31502
Monascus ruber strain BCRC 31532
Monascus pilosus of the present invention ITS
Monascus pilosus strain ATCC 16364
Monascus ruber strain ATCC 58358
Monascus pallens strain IMI356820
Monascus floridanus strain BCRC33310
Monascus eremophilus strain ATCC 62925
Monascus lunisporas strain ATCC 204397
Monascus argentinensis strain BCRC 33998
Aspergillus nidulans ATCC 10074
Aspergillus fumigatus ATCC 1022
Aspergillus niger ATCC 16888
Aspergillus terreus ATCC 1012
Aspergillus oryzae NRRL 447
Aspergillus japonicus strain CBS 114.51
99
84
62
37
24
90
91
99
56
35
97
39
57
47
0.050


FIG. 3

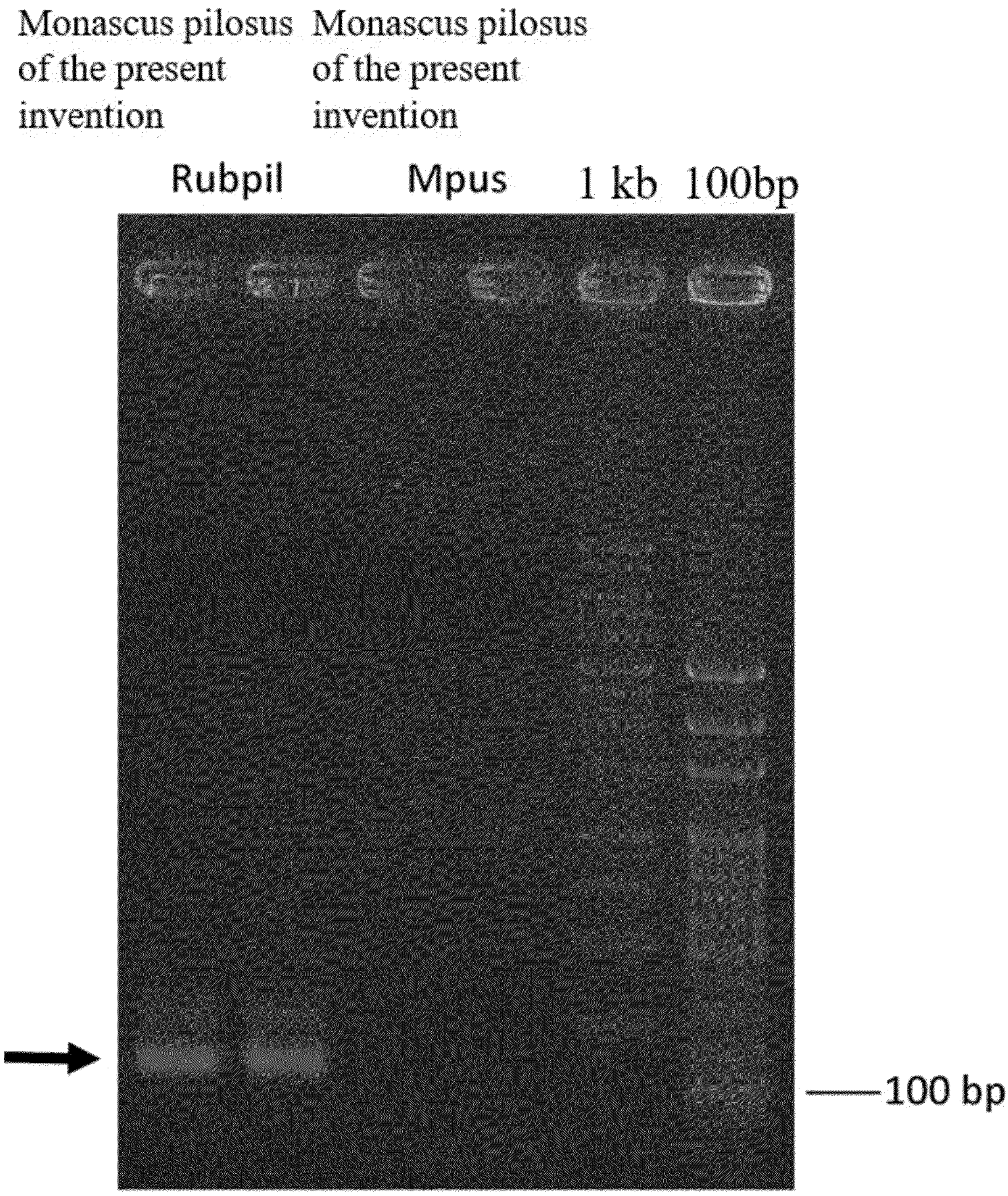
Monascus pilosus of the present invention
Monascus pilosus of the present invention
Rubpil
Mpus
1 kb
100bp
100 bp

FIG. 4

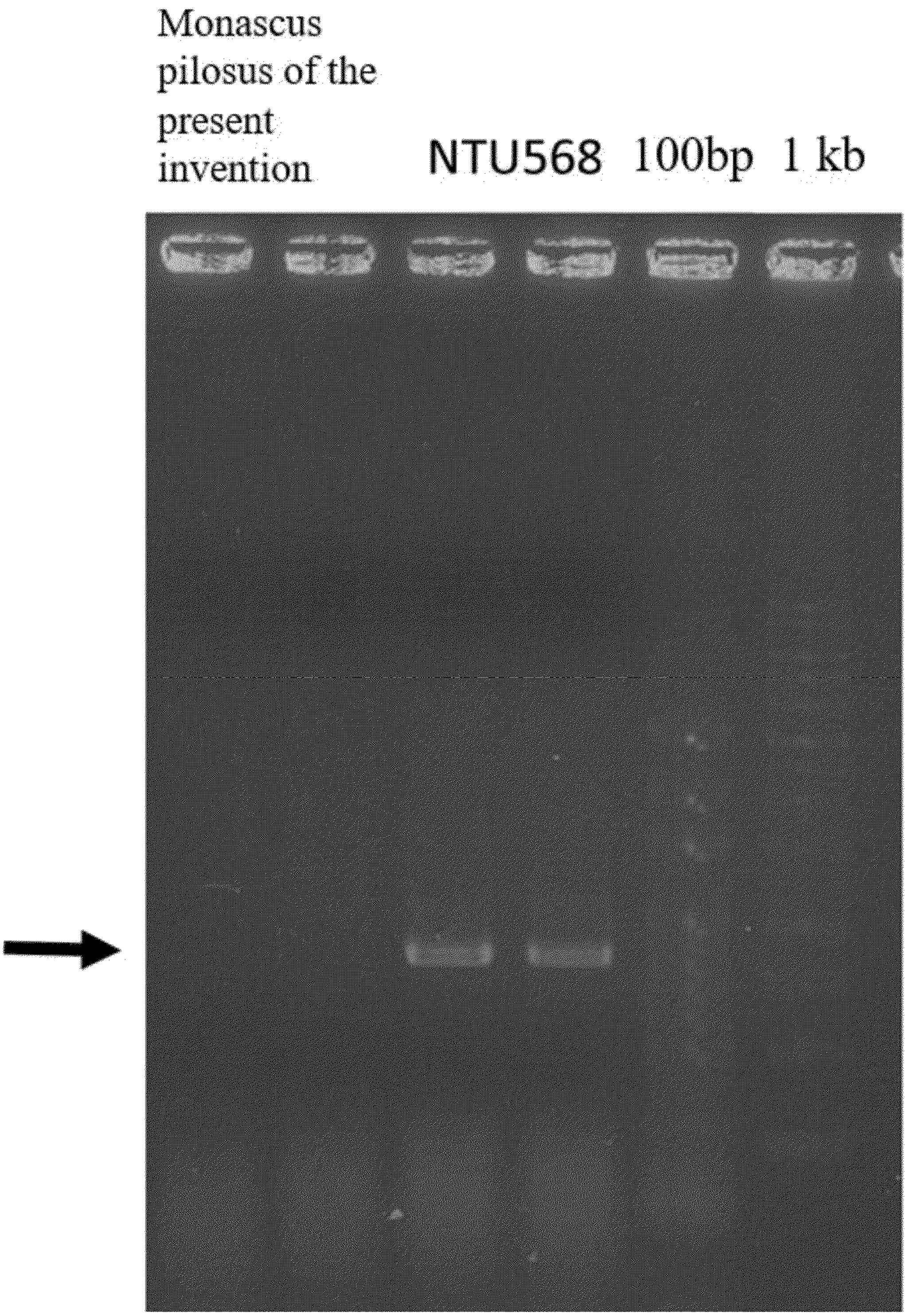
Monascus pilosus of the present invention
NTU568
100bp
1 kb

FIG. 5

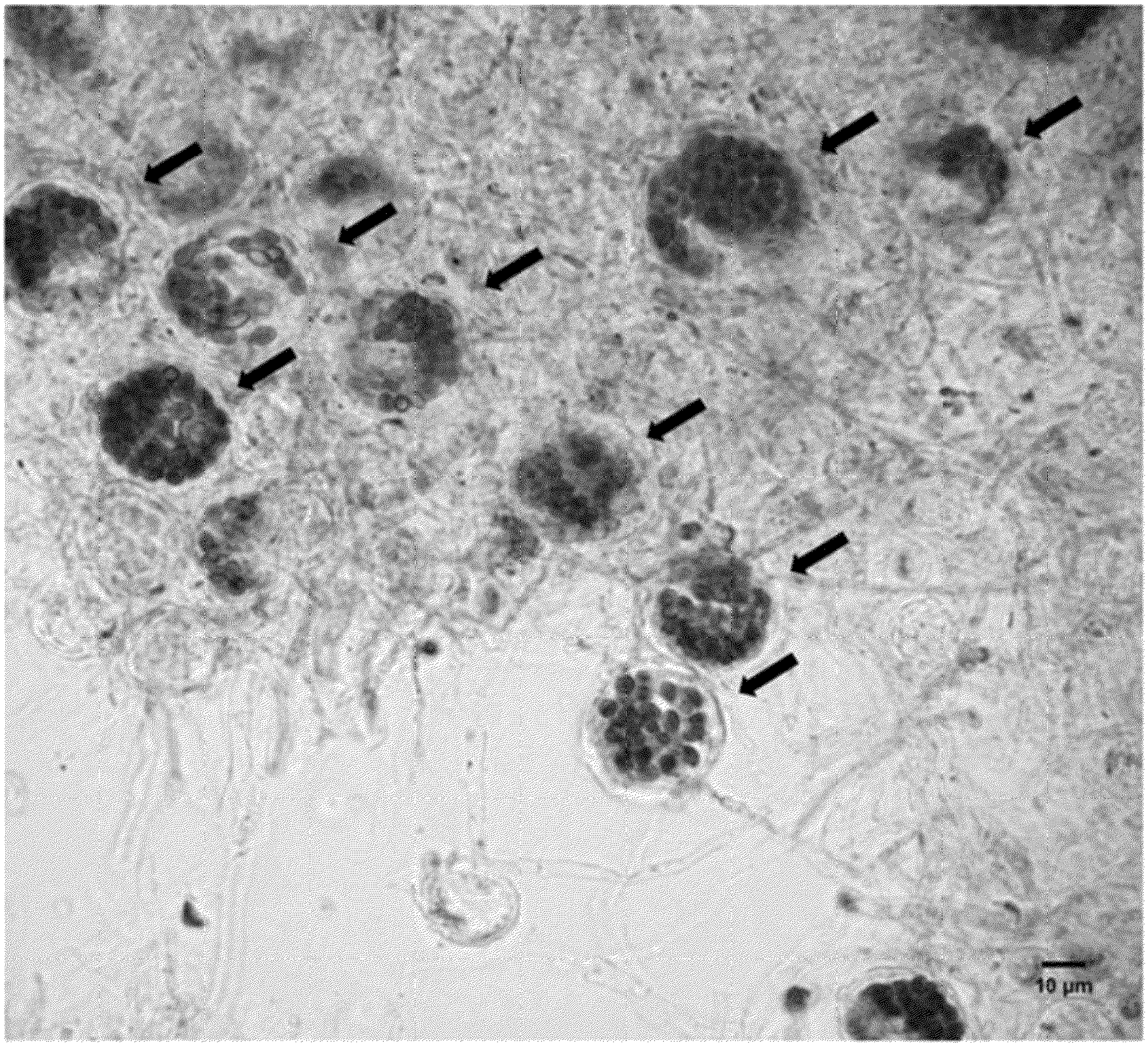


FIG. 6

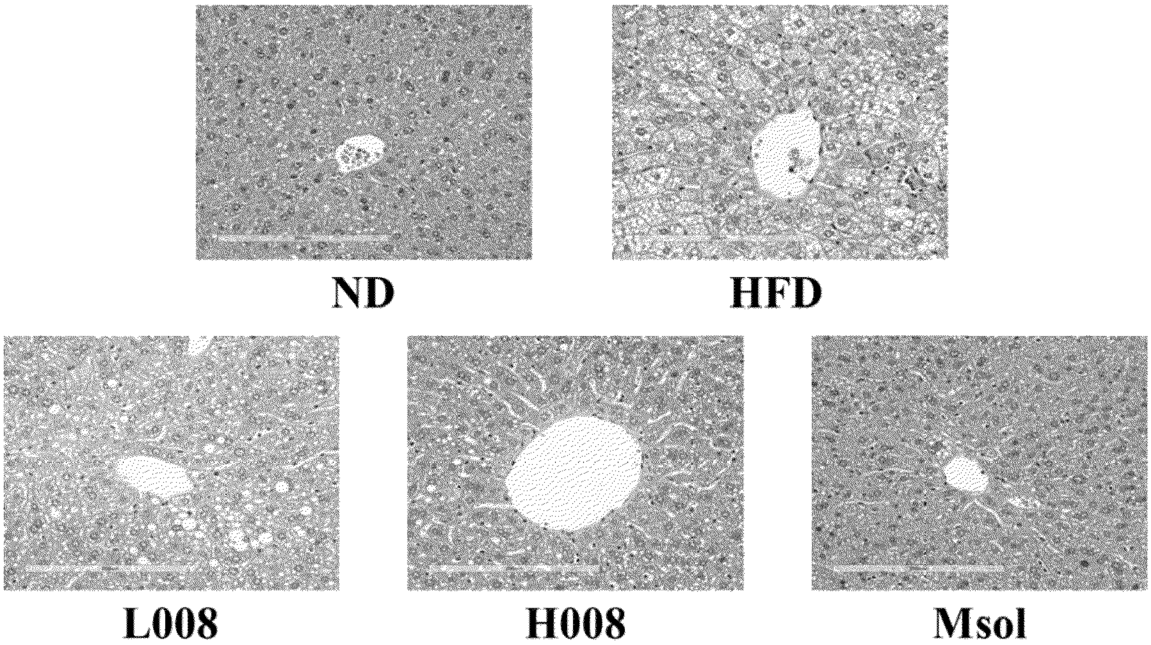


FIG. 7

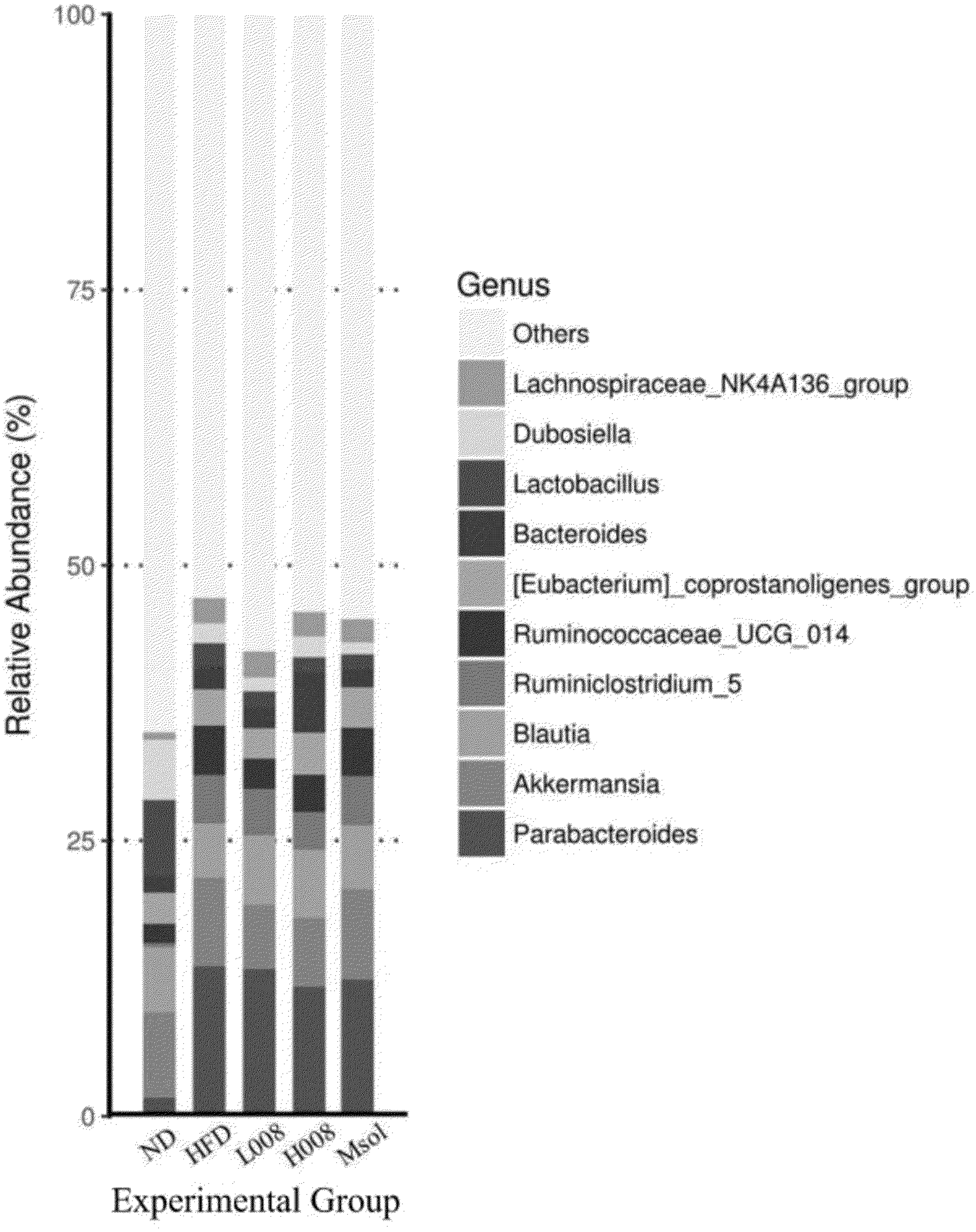

100
75
50
25
0
Relative Abundance (%)
ND
HFD
L008
H008
Msol
Experimental Group
Genus
Others
Lachnospiraceae_NK4A136_group
Dubosiella
Lactobacillus
Bacteroides
[Eubacterium]_coprostanoligenes_group
Ruminococcaceae_UCG_014
Ruminiclostridium_5
Blautia
Akkermansia
Parabacteroides


FIG. 8

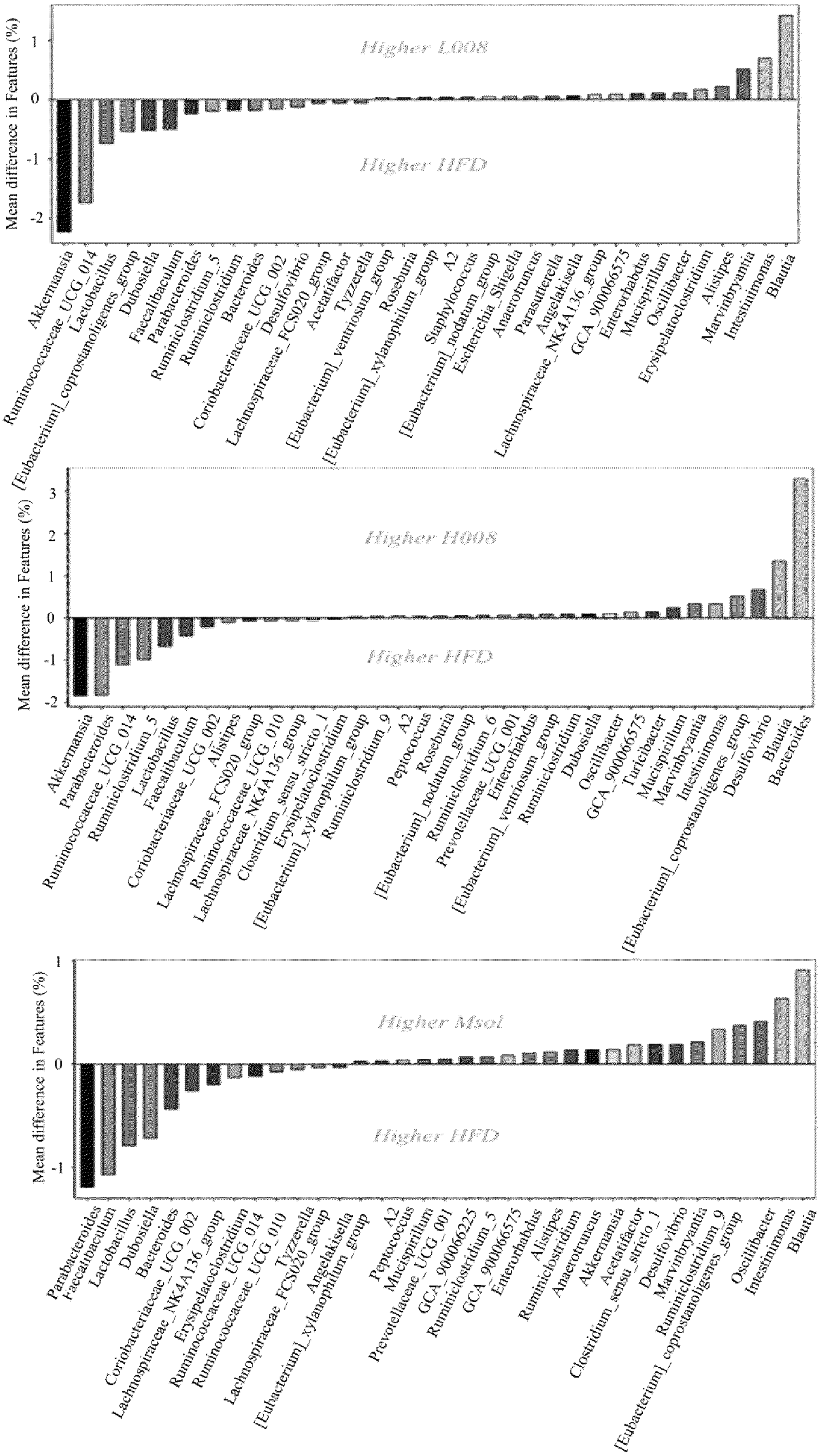
Mean difference in Features (%)
Higher L008
Higher HFD
Akkermansia
Ruminococcaceae_UCG_014
Lactobacillus
[Eubacterium]_coprostanoligenes_group
Dubosiella
Faecalibaculum
Parabacteroides
Ruminiclostridium_5
Ruminiclostridium
Bacteroides
Coriobacteriaceae_UCG_002
Desulfovibrio
Lachnospiraceae_FCS020_group
Acetatifactor
Tyzzerella
[Eubacterium]_ventriosum_group
Roseburia
[Eubacterium]_xylanophilum_group
A2
Staphylococcus
[Eubacterium]_nodatum_group
Escherichia_Shigella
Anaerotruncus
Parasutterella
Angelakisella
Lachnospiraceae_NK4A136_group
GCA_900066575
Enterorhabdus
Mucispirillum
Oscillibacter
Erysipelatoclostridium
Alistipes
Marvinbryantia
Intestinimonas
Blautia
Higher H008
Higher HFD
Akkermansia
Parabacteroides
Ruminococcaceae_UCG_014
Ruminiclostridium_5
Lactobacillus
Faecalibaculum
Coriobacteriaceae_UCG_002
Alistipes
Lachnospiraceae_FCS020_group
Ruminococcaceae_UCG_010
Lachnospiraceae_NK4A136_group
Clostridium_sensu_stricto_1
Erysipelatoclostridium
[Eubacterium]_xylanophilum_group
Ruminiclostridium_9
A2
Peptococcus
Roseburia
[Eubacterium]_nodatum_group
Ruminiclostridium_6
Prevotellaceae_UCG_001
Enterorhabdus
[Eubacterium]_ventriosum_group
Ruminiclostridium
Dubosiella
Oscillibacter
GCA_900066575
Turicibacter
Mucispirillum
Marvinbryantia
Intestinimonas
[Eubacterium]_coprostanoligenes_group
Desulfovibrio
Blautia
Bacteroides
Higher Msol
Higher HFD
Parabacteroides
Faecalibaculum
Lactobacillus
Dubosiella
Bacteroides
Coriobacteriaceae_UCG_002
Lachnospiraceae_NK4A136_group
Erysipelatoclostridium
Ruminococcaceae_UCG_014
Ruminococcaceae_UCG_010
Tyzzerella
Lachnospiraceae_FCS020_group
Angelakisella
[Eubacterium]_xylanophilum_group
A2
Peptococcus
Mucispirillum
Prevotellaceae_UCG_001
GCA_900066225
Ruminiclostridium_5
GCA_900066575
Enterorhabdus
Alistipes
Ruminiclostridium
Anaerotruncus
Akkermansia
Acetatifactor
Clostridium_sensu_stricto_1
Desulfovibrio
Marvinbryantia
Ruminiclostridium_9
[Eubacterium]_coprostanoligenes_group
Oscillibacter
Intestinimonas
Blautia

FIG. 9

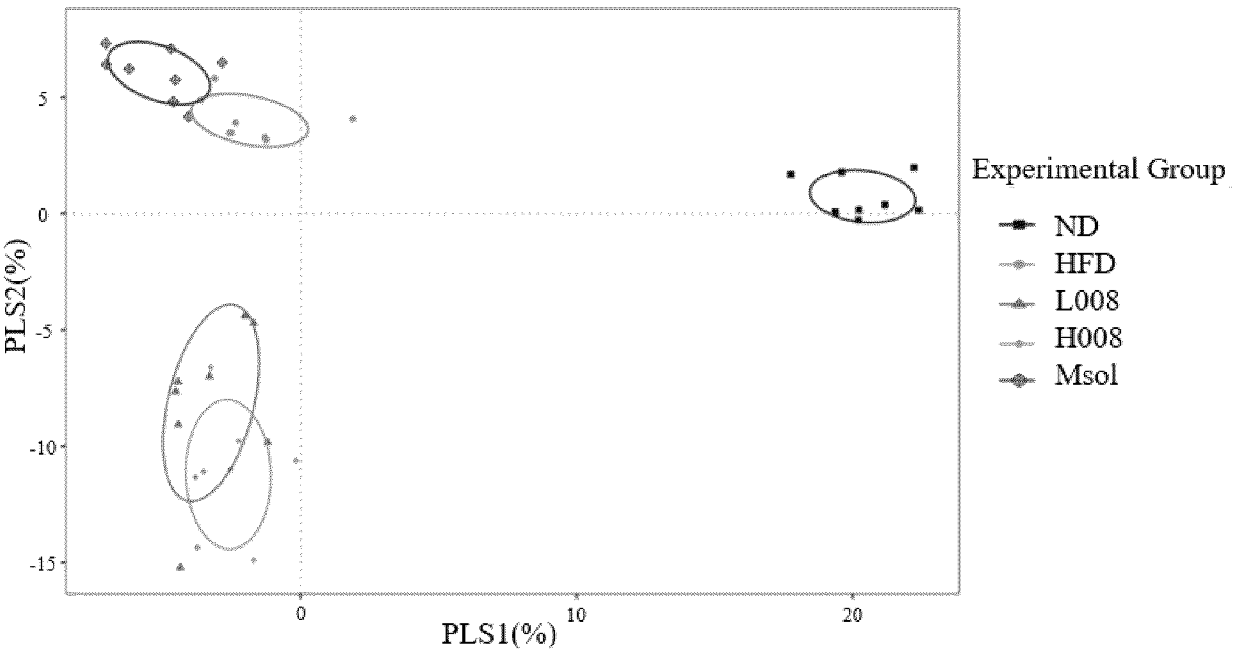
5
0
-5
-10
-15
PLS2(%)
0
10
20
PLS1(%)
Experimental Group
ND
HFD
L008
H008
Msol

FIG. 10

Europäisches Patentamt
European Patent Office
Office européen des brevets

# EUROPEAN SEARCH REPORT

Application Number
EP 24 20 2826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HSU WEI-HSUAN ET AL: "Monascin and ankaflavin act as natural AMPK activators with PPAR[alpha] agonist activity to down-regulate nonalcoholic steatohepatitis in high-fat diet-fed C57BL/6", FOOD AND CHEMICAL TOXICOLOGY, PERGAMON, GB, vol. 64, 22 November 2013 (2013-11-22), pages 94-103, XP028810434, ISSN: 0278-6915, DOI: 10.1016/J.FCT.2013.11.015 * page 95, column 1, paragraph 3 * ----- | 1-14 | INV. A61K31/352 A61P1/16 |
| A | CN 117 964 637 A (SHANGHAI YIDI BIOTECHNOLOGY CO LTD) 3 May 2024 (2024-05-03) * claim 7; compound formula (II) * * claim 8; compound 22 * ----- | 1-14 | |
| | | | TECHNICAL FIELDS SEARCHED (IPC) |
| | | | A61K A61P |

~~The present search report has been drawn up for all claims~~

2

| Place of search | Date of completion of the search | Examiner |
|---|---|---|
| The Hague | 11 February 2025 | Uryga-Polowy, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 24 20 2826

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 20 2826

The Search Division considers that the present European patent application does not comply with the requirements of unity of invention and relates to several inventions or groups of inventions, namely:

1. claims: 1-14(partially)

   compounds of formula (I) where R2 is C, compositions thereof, per se and for use in preventing or treating nonalcoholic fatty liver disease

   ---

2. claims: 1-14(partially)

   compounds of formula (I) where R2 is O and R1 is alkylcarbonyl, compositions thereof, per se and for use in preventing or treating nonalcoholic fatty liver disease

   ---

3. claims: 1-14(partially)

   compounds of formula (I) where R2 is O and R1 is hydroxyalkyl, compositions thereof, per se and for use in preventing or treating nonalcoholic fatty liver disease

   ---

# ANNEX TO THE EUROPEAN SEARCH REPORT ON EUROPEAN PATENT APPLICATION NO. EP 24 20 2826

This annex lists the patent family members relating to the patent documents cited in the above-mentioned European search report. The members are as contained in the European Patent Office EDP file on
The European Patent Office is in no way liable for these particulars which are merely given for the purpose of information.

11-02-2025

| Patent document cited in search report | Publication date | Patent family member(s) | Publication date |
|---|---|---|---|
| CN 117964637 A | 03-05-2024 | NONE | |

EPO FORM P0459

For more details about this annex : see Official Journal of the European Patent Office, No. 12/82